# FASCICULE DE BREVET EUROPEEN

(11) **EP 0 834 492 B1**
(45) Date de publication et mention de la délivrance du brevet: **28.12.2005**
(21) Numéro de dépôt: 97402312.9
(22) Date de dépôt: 02.10.1997
(51) Int. Cl.: C07C 25/18, C07C 311/48, C07C 381/12, C07F 17/00, C08F 220/12, C08F 230/04, C08G 77/24, G03F 7/029, G03F 7/039, C08F 2/46

(54) **Sulfonylimidures et sulfonylméthylures ioniques fluorés, leur procédé de préparation et leur utilisation comme photoamorceurs**
Fluorierte ionische Sulfonylimide und Sulfonylmethylide, Verfahren zu deren Herstellung sowie deren Anwednung als Photoinitiatoren
Fluorinated ionic sulphonylimides and sulphonylmethylides, process for their preparation and their use as photoinitiators

(30) Priorité: 03.10.1996 CA 2187046
(43) Date de publication de la demande: 08.04.1998
(73) Titulaire: HYDRO-QUEBEC, Montréal Québec H2Z 1A4 (CA); CENTRE NATIONAL DE LA RECHERCHE SCIENTIFIQUE (CNRS), 75016 Paris (FR)
(72) Inventeur: Vallee, Alain, Varennes, Québec, J3X 1Y9 (CA); Armand, Michel, Montréal, Québec, H3T 1N2 (CA); Ollivrin, Xavier, 38000 Grenoble (FR); Michot, Christophe, 38000 Grenoble (FR)
(74) Mandataire: Sueur, Yvette

(56) Documents cités:
- EP-A- 0 703 236
- US-A- 4 197 174
- US-A- 4 780 511
- US-A- 5 340 898
- US-A- 5 550 171
- US-A- 5 554 664

## Description

La présente invention concerne des composés ioniques, un procédé pour leur préparation, et leur utilisation comme photoamorceurs pour la polymérisation ou la réticulation par voie cationique de monomères et de prépolymères, ou pour la modification des paramètres de solubilité de certains polymères qui peuvent être utilisés comme photorésists.

Une polymérisation mettant en jeu un mécanisme de type cationique présente de nombreux avantages. En particulier, elle est rapide, même à basse température, le taux d'utilisation du monomère est élevé et la sensibilité aux contaminants atmosphériques, notamment l'oxygène, est faible par comparaison aux polymérisations radicalaires ou anioniques.

Les monomères, les prépolymères et les polymères contenant des fonctions époxydes cycloaliphatiques et les éthers vinyliques sont utilisés de plus en plus notamment dans l'industrie des peintures, des vernis, des encres, des colles et des supports anti-adhésifs. De plus, les éthers vinyliques apparaissent d'une manière générale dénués de toxicité, contrairement aux acrylates ou aux méthacrylates. Les monomères et les prépolymères du type époxydes ou du type éther vinylique peuvent être polymérisés selon différentes méthodes, la polymérisation cationique étant particulièrement intéressante.

Les catalyseurs de polymérisation cationique sont d'une manière générale des acides au sens de Brønsted HX (donneurs de protons), ou des acides au sens de Lewis (accepteurs de doublets électroniques), ces derniers fonctionnant en présence d'un co-catalyseur source de protons. Il est nécessaire que ces acides soient suffisamment forts pour assurer la stabilité de l'espèce cationique portée soit par le monomère, soit par la chaîne macromoléculaire en croissance, ce qui signifie que l'anion correspondant X⁻ doit posséder le pouvoir nucléophile le plus faible possible. Les acides de Brønsted les plus utilisés comme catalyseurs de polymérisation cationique sont CF₃SO₃H, HClO₄, HBF₄, HPF₆, HAsF₆ et SbF₆. Ces acides se classent comme suit, en ce qui concerne les vitesses d'amorçage, de propagation ainsi que l'obtention des masses moléculaires les plus élevées :

CF₃SO₃H < HClO₄ ≈ HBF₄ < HPF₆ ≈ HAsF₆ ≈ SbF₆.

Plus récemment, on a également utilisé des composés à caractère acide tels que le bis(perfluoroalkylsulfonyl)-imide (US-A-4,031,036, Koshar, et al) ou le bis(perfluoroalkylsulfonyl)méthane (US-A-3,632,843, Allen et al).

Il est connu que la préparation in situ de catalyseurs de polymérisation présente de nombreux avantages. La production in situ de l'acide capable de catalyser la réticulation d'un monomère permet en effet de mettre en forme un monomère ou un prépolymère fluide (matériau thermoplastique ou solution) et de lui donner ses propriétés définitives par exemple par simple action d'un rayonnement. Cette technique est très utilisée pour les encres, les peintures, les films adhésifs et les films anti-adhésifs. Il faut noter en outre que la préparation de l'acide in situ à partir d'un sel permet dans de nombreux cas d'éviter le stockage et la manipulation de composés acides plus corrosifs que les sels correspondants.

Les catalyseurs peuvent être préparés in situ par action de chaleur. Par exemple, des sels d'ammonium ou de métal de bis(perfluoroalkylsulfonyl)imide (US-A-4,031,036, Koshar, et al) ou des sels d'ammonium ou d'amine de bis-(perfluoroalkylsulfonyl)méthane (US-A-3,632,843, Allen et al) ont été utilisés pour obtenir in situ, par chauffage, le bis(perfluoroalkylsulfonyl)imide ou le bis(perfluoroalkylsulfonyl)méthane correspondant, qui agit ensuite comme catalyseur. Ces catalyseurs, dits "latents", ne présentent cependant qu'un intérêt limité du fait de la nécessité d'un chauffage prolongé à température élevée pour obtenir la libération de l'acide, cette libération étant en outre progressive, et non pas intégrale à l'initiation. Il en résulte d'une part une faible vitesse de réaction, et d'autre part des polymères de qualité médiocre en ce qui concerne la masse moléculaire, la polydispersité et la coloration.

Les catalyseurs acides peuvent également être préparés in situ par action d'un rayonnement actinique (tel que les photons dont la longueur d'onde correspond au rayonnement ultraviolet, visible, γ et X), ou d'un rayonnement β (faisceau d'électrons) sur un sel approprié. Un tel sel, qui présente une labilité chimique sous l'action d'un rayonnement actinique ou d'un rayonnement β amenant la libération de l'acide correspondant ayant une forte activité catalytique, est un photoamorceur. Les avantages d'un tel procédé sont multiples : la libération du catalyseur par irradiation est rapide et pratiquement totale, ce qui entraîne une initiation simultanée de la croissance des chaînes, et donc un répartition des masses plus homogène avec une moindre polydispersité, et de meilleures propriétés mécaniques. La polymérisation peut être effectuée à une température relativement basse, ce qui évite une décomposition ou une coloration des matériaux obtenus, ainsi que la formation de bulles lorsqu'un solvant est utilisé ou lorsque le mélange réactionnel contient un additif volatil que l'on souhaite conserver dans le matériau final et qui joue le rôle de plastifiant.

US-A-5,554,664 décrit des sels activables sous l'effet d'énergie dans lesquels le cation comprend au moins un cation choisi parmi les cations organo-métalliques comprenant un composé aromatique basé sur un arène ou un ligand pentadiényle et un métal de transition, et parmi les cations iodonium, les cations sulfonium, les cations phosphonium et les cations carbonium comprenant un métal de transition, et dans lesquels le nombre d'anions est suffisant pour neutraliser la charge du cation, l'anion étant un tris(alkylsulfonylméthylure), un bis(alkylsulfonylimidure), un tris(arylsulfonylméthylure) ou un bis(arylsulfonylimidure) dans lesquels le groupe alkyle ou le groupe aryle est éventuellement perfluoré ou fortement fluoré. Les sels d'un simple métal, les sels de diazonium et les sels d'ammonium sont exclus. Ces sels peuvent être utilisés comme photoamorceur pour la polymérisation cationique d'oléfines.

Il est également connu d'utiliser des acides générés à l'aide d'un rayonnement actinique pour dégrader les résines contenues dans un film constituant un photorésist. Cette technique est particulièrement efficace pour les photorésists à amplification chimique, dans lesquels de très faibles quantités de protons catalysent la décomposition de groupements tels que des esters contenant un groupement dérivé d'un alcool tertiaire (tel que par exemple un groupement tertiobutyle), faisant partie d'une chaîne macromoléculaire. Elle permet ainsi de modifier les paramètres de solubilité de la résine exposée à un rayonnement actinique et d'effectuer des opérations de masquage et de gravure sélectives telles qu'utilisées en microélectronique.

Les inventeurs ont maintenant trouvé de nouveaux composés ioniques qui permettent d'obtenir sous l'action d'un rayonnement actinique ou d'un rayonnement β, des acides qui se révèlent bons catalyseurs de polymérisation cationique ou de modification de polymères.

La présente invention a ainsi pour objet une nouvelle famille de composés ioniques, un procédé pour leur préparation, ainsi que leur utilisation comme photoamorceurs pour la polymérisation ou la réticulation de monomères par voie cationique, ou pour la modification de polymères, notamment lorsqu'ils sont utilisés comme photorésists.

Un composé ionique de la présente invention contient au moins un groupement A⁺X⁻, et il est caractérisé en ce que :
- A⁺ est un groupe cationique choisi dans le groupe constitué par les groupements biaryliodonium, les groupements arylsulfonium, les groupements arylacylsulfonium, les groupements diazonium et les cations organométalliques comprenant un métal de transition complexé par au moins un cycle insaturé comprenant de 4 à 12 atomes de carbone, ledit groupe cationique faisant éventuellement partie d'une chaîne polymère ;
- X⁻ est un anion imidure [FSO₂NSO₂R'_{F}]⁻ ou [R_{F}CH₂OSO₂NSO₂R'_{F}]⁻ ou [(R_{F})₂CHOSO₂NSO₂R'_{F}]⁻, ou un anion méthylure [FSO₂C(Q)SO₂R'_{F}]⁻ ou [R_{F}CH₂OSO₂C(Q)SO₂R'_{F}]⁻ ou [(R_{F})₂CHOSO₂C(Q)SO₂R'_{F}]⁻ dans lesquels :
   1) Q représente :
      - H-, Cl-, F-, Br- ou CN- ;
      - un radical alkyle ayant de 1 à 30 atomes de carbone ;
      - un radical aryle ou alkylaryle ou arylalkyle ayant de 6 à 30 atomes de carbone ;
      - un groupe R"_{F}-, un groupe R"_{F}SO₂-, un groupe R"_{F}CH₂O-SO₂- ou un groupe (R"_{F})₂CHO-SO₂- ;
   2) R_{F} et R'_{F}, ainsi que R"_{F} le cas échéant lorsque X⁻ est un anion méthylure, sont choisis indépendamment l'un de l'autre dans le groupe constitué par le fluor, par les groupements perhaloalkyles ayant de 1 à 30 atomes de carbone, par les groupements (perhaloalkyl)alcoxy ayant de 2 à 30 atomes de carbone, par les groupements cycloaliphatiques perhalogénés ayant de 3 à 30 atomes de carbone contenant éventuellement des hétéroatomes choisis parmi O et N et/ou portant éventuellement au moins un chaînon perhaloalkyle, et par les groupements aryles perhalogénés ayant de 6 à 30 atomes de carbone; ou bien
   3) R_{F} et R'_{F} forment ensemble un radical divalent choisi parmi les radicaux alkylènes linéaires perfluorés ayant de 2 à 8 atomes de carbone ; ou bien
   4) lorsque X⁻ est un anion méthylure, R'_{F} et R"_{F} d'une part, ou R_{F} et R"_{F} d'autre part forment ensemble un radical divalent choisi parmi les radicaux alkylènes linéaires perfluorés ayant de 2 à 8 atomes de carbone.

Comme exemples particulièrement intéressants d'anions X⁻, on peut citer les anions sulfonimidures [FSO₂NSO₂R'_{F}]⁻, [CF₃CH₂OSO₂NSO₂R'_{F}]⁻ et [(CF₃)₂CHOSO₂NSO₂R'_{F}]⁻, dans lesquels le radical R'_{F} est F, CF₃CH₂O-, (CF₃)₂CH-O- ou un groupement perfluoroalkyle ayant de 1 à 10 atomes de carbone (de préférence CF₃-, C₂F₅-, C₄F₉-, C₆F₁₃-, C₈F₁₇- et C₁₀F₂₁-).

On peut également citer les anions sulfonylméthylures [FSO₂C(Q)SO₂R'_{F}]⁻, [CF₃CH₂OSO₂C(Q)SO₂R'_{F}]⁻, [(CF₃)₂CHOSO₂-C(Q)SO₂R'_{F}]⁻, dans lesquels R'_{F} est F, CF₃CH₂O-, (CF₃)₂CH-O- ou un groupement perfluoroalkyle ayant de 1 à 10 atomes de carbone (de préférence CF₃-, C₂F₅-, C₄F₉-, C₆F₁₃-, C₈F₁₇- et C₁₀F₂₁-) ; Q est choisi dans le groupe constitué par l'hydrogène, FSO₂-, CF₃CH₂O-SO₂-, (CF₃)₂CH-O-SO₂-, les groupements alkyles, aryles, alkylaryles ou arylalkyles ayant au plus 30 atomes de carbone, les groupements perfluoroalkylsulfonyles ayant de 1 à 8 atomes de carbone (de préférence CF₃SO₂-, C₂F₅SO₂-, C₄F₉SO₂-, C₆F₁₃SO₂- et C₈F₁₇SO₂-) et les groupements perfluoroalkyles ayant de 1 à 12 atomes de carbone (de préférence CF₃-, C₂F₅-, C₄F₉-, C₆F₁₃-, C₈F₁₇- et C₁₀F₂₁-) ; ou bien Q et R'_{F} forment ensemble un groupement divalent alkylène linéaire perfluoré ayant de 1 à 8 atomes de carbone.

Parmi les anions définis ci-dessus, ceux qui font partie du groupe constitué par (FSO₂)₂N⁻, (FSO₂)₃C⁻, (FSO₂)₂CH⁻, (CF₃CH₂OSO₂)₂N⁻, [(CF₃)₂CHOSO₂]₂N⁻, (CF₃CH₂OSO₂)₂CH⁻, [(CF₃)₂-CHOSO₂]₂CH⁻, [(CF₃)₂CHOSO₂]₃C⁻, [(CF₃)₂CHOSO₂]₃C⁻, [FSO₂NSO₂-CF₃]⁻, [FSO₂NSO₂C₂F₅]⁻, [(CF₃)₂CHO-SO₂NSO₂CF₃]⁻, [(CF₃)₂CHOSO₂₋ NSO₂CF₃]⁻, [(CF₃)₂CHOSO₂NSO₂C₂F₅]⁻ et [(CF₃CH₂OSO₂NSO₂C₂F₅]⁻ sont particulièrement intéressants.

Un composé selon l'invention peut se présenter sous la forme d'un monomère. Il se définit alors comme un composé A⁺X⁻ dans lequel X est tel que défini précédemment et le cation A⁺ répond à la formule 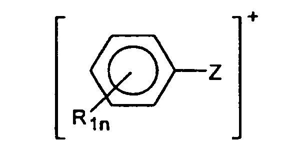 dans laquelle Z est choisi dans le groupe constitué par :
- les groupements aryliodonium 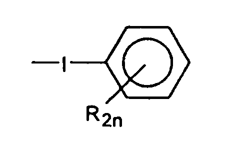
- les groupements sulfonium 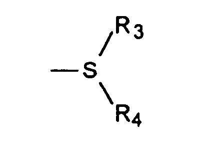
- les groupements acylsulfonium 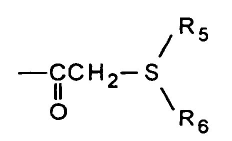
- les groupements diazonium

   N≡N-R₈
- les groupements organométalliques 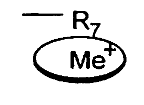 et dans laquelle R₁ₙ représente de 1 à 4, de préférence 1 à 2 groupements identiques ou différents liés à l'un quelconque des atomes de carbone libres du groupe aryle, R₂ₙ représente de 1 à 4, de préférence de 1 à 2 groupements identiques ou différents liés à l'un quelconque des atomes de carbone libres du groupe aryle, et les groupements R₁ₙ, R₂ₙ, et R₃ à R₈ sont choisis indépendamment les uns des autres parmi :
   - les radicaux alkyles ou arylalkyles linéaires ou ramifiés ayant de 1 à 30 atomes de carbone ;
   - les radicaux alkényles ayant de 1 à 30 atomes de carbone ;
   - les radicaux aryles ou alkylaryles ayant de 6 à 30 atomes de carbone, incluant ceux qui ont des noyaux condensés ;
   - les radicaux ayant de 1 à 30 atomes de carbone et choisis dans le groupe constitué par les oxaalkyles, les azaalkyles, les thiaalkyles, les phosphaalkyles, les oxaalkylènes, les azaalkylènes, les thiaalkylènes, les phosphaalkylènes,
   - les radicaux ayant de 1 à 30 atomes de carbone et incluant un groupement sulfoxyde, un groupement sulfone, un groupement oxyde de phosphine, un groupement phosphonate, tous ces radicaux étant obtenus par addition d'oxygène sur les atomes de soufre ou de phosphore ;
   - les radicaux hétérocycliques aromatiques ou alicycliques comprenant au moins un hétéroatome choisi dans le groupe constitué par O, N, S et P ;
   - -NO, -CN, -OH, -Cl, -Br, -I, -F ;
   - ou bien deux substituants choisis parmi les R₁ₙ et/ou deux substituants choisis parmi les R₂ₙ, ou les substituants R₃ et R₄, ou les substituants R₅ et R₆ forment ensemble un radical divalent qui forme un cycle avec le groupement qui les porte, ledit radical divalent étant choisi dans le groupe constitué par les radicaux alkylènes linéaires ayant de 1 à 18 atomes de carbone, les biradicaux benzo portant éventuellement au moins un substituant choisi de préférence dans le groupe constitué par les radicaux alkyles, oxaalkyles ou alcényles ayant de 1 à 10 atomes de carbone, les groupements oxaalkylènes répondant à la formule -R'-(OCH₂CH₂)_{q}-OR'- ou -R'-[OCH(CH₃)CH₂]_{q}-OR'- dans lesquels R' est H ou un radical alkylène linéaire ayant de 1 à 18 atomes de carbone et 1≤q≤22.

Les composés monomères préférés sont ceux dans lesquels les substituants R₁ₙ, R₂ₙ, et R₃ à R₈ sont choisis indépendamment les uns des autres dans le groupe constitué par les radicaux alkyles linéaires ayant de 1 à 18 atomes de carbone, le 2-éthylhexyle, le phényle, les oxaalkyles répondant à la formule R-(OCH₂CH₂)_{y}- ou R-[OCH(CH₃)CH₂]_{y}- dans lesquels R est H ou un radical alkyle linéaire ayant de 1 à 18 atomes de carbone et 1≤y≤22.

Les composés ioniques A⁺X⁻ de la présente invention sont en général insolubles dans l'eau. Ils peuvent donc être préparés par un procédé consistant à effectuer une métathèse dans l'eau ou dans un mélange eau/alcool léger (méthanol, éthanol, propanol) entre un sel soluble A⁺X₁⁻ dans lequel X₁⁻ est un anion de caractère hydrophile, et un composé A₁⁺X⁻ soluble dans l'eau, A₁⁺ étant un cation fortement hydrophile.

Les sels solubles A⁺X₁⁻ dans lesquels A⁺ représente un cation iodonium, sulfonium, acylsulfonium, diazonium ou un cation organométallique tel que défini ci-dessus, sont choisis de préférence parmi les sels dans lesquels l'anion X₁⁻ est un hydroxyde, un chlorure, un bromure, un hydrogénosulfate, un dihydrogénophosphate ou un méthylsulfonate. Ces anions étant fortement solvatés par l'eau ou les alcools légers, ils favorisent la solubilité.

Les composés A₁⁺X⁻ solubles dans l'eau ou dans les mélanges alcool/eau sont choisis de préférence parmi les sulfonimides et les sulfonylméthanes de sodium, de potassium, d'ammonium, de calcium ou de magnésium. Le choix du cation dépend bien entendu de la facilité d'obtention et du minimum d'hydrophilie requise pour entraîner la solubilité.

Lorsqu'un composé A⁺X⁻ de l'invention est préparé à partir d'un sel A⁺X₁⁻ dans lequel X₁ est un chlorure, un bromure, un alkylsulfonate, un alkyloxysulfonate ou un arylsulfonate, dont les sels de sodium NaX₁ ou de potassium KX₁ sont insolubles dans les solvants usuels, il est avantageux de conduire la réaction en présence d'un sel NaX ou KX. La solubilité de ces sels dans les solvants même de moyenne polarité étant appréciable, les sels insolubles tel que NaCl, KBr précipitent alors que le composé ionique de l'invention reste en solution. Comme solvants, on peut citer l'acétone, le méthyléthylcétone, l'acétonitrile, le THF, les esters tels que les formiates ou l'acétate de méthyle ou d'éthyle.

Il faut noter que tout autre procédé d'échange ionique peut être mis en oeuvre, par exemple un procédé utilisant une résine échangeuse d'ions, ou un procédé de précipitation sélective.

Les inventeurs ont trouvé de manière surprenante que les composés possédant des groupements fluorosulfonyles FSO₂ étaient stables en présence de cations sensibles au rayonnement actinique et que les acides libérés étaient actifs pour amorcer des réactions de polymérisation ou de réticulation par l'intermédiaire d'espèces cationiques. De plus, les anions dont la charge négative est délocalisée sur les groupement FSO₂ sont apparus comme susceptibles d'induire des solubilités remarquables des sels correspondants dans des solvants usuels, comparables à celles obtenues avec les groupements perfluoroalkyles. Les anions contenant un ou plusieurs groupements FSO₂ en remplacement des groupements perfluoroalkyles ont cependant un poids moléculaire moindre et sont donc actifs à des fractions pondérales plus faibles. Leur coût est plus avantageux, ne faisant pas appel à des méthodes de fluoration chimique qui ont de faibles rendements. Les mêmes propriétés de solubilité et d'activité en polymérisation ont été observées avec les composés contenant les groupements R_{F}-CH₂O-SO₂- et (R_{F})₂CHO-SO₂-, R_{F} représentant un groupement fluoroalkyle. De manière surprenante, la liaison carbone-oxygène d'un groupement R_{F}-CH₂O-SO₂- ou (R_{F})₂CHO-SO₂- est stable à l'hydrolyse, contrairement à ce qui est observé avec les esters sulfoniques hydrocarbonés (R-CH₂O-SO₂). En outre, les anions comprenant les groupements R_{F}-CH₂O-SO₂- et (R_{F})₂CHO-SO₂- sont facilement obtenus à partir des dérivés -SO₂F ou -SO₂Cl par action des alcools halogénés correspondants R_{F}CH₂OH et (R_{F})₂CHOH qui sont produits industriellement. Comparés aux composés analogues ayant des groupements CF₃SO₂-, les composés contenant un groupement FSO₂- sont moins corrosifs vis-à-vis des métaux comme l'aluminium car ils forment une couche protectrice de fluorure, ce qui est un élément important en technologie des peintures. Il apparaît que les groupements R_{F}CH₂OSO₂- et (R_{F})₂CHOSO₂- ont un comportement analogue à celui des groupements FSO₂-.

La présente invention a par conséquent également pour objet l'utilisation des composés ioniques de l'invention comme photoamorceurs sources d'acides de Brønsted catalyseurs de polymérisation ou de réticulation de monomères ou de prépolymères capables de réagir par voie cationique, ou catalyseurs pour la modification de polymères. Le procédé de polymérisation ou de réticulation de monomères ou de prépolymères capables de réagir par voie cationique est caractérisé en ce que l'on utilise un composé de l'invention comme photoamorceur source d'acide catalysant la réaction de polymérisation.

Lorsque le composé A⁺X⁻ de l'invention est destiné à être utilisé comme photoamorceur pour la polymérisation de monomères ou de prépolymères polymérisant par voie cationique, le choix des groupements R₁ à R₈ est effectué parmi les radicaux ci-dessus pour augmenter la solubilité dudit composé dans les solvants de mise en oeuvre des monomères ou des prépolymères et en fonction des propriétés souhaitées pour le polymère final. Par exemple, le choix de radicaux alkyles non substitués donne une solubilité dans les milieux peu polaires. Le choix de radicaux comprenant un groupe oxa ou une sulfone donne une solubilité dans les milieux polaires. Les radicaux incluant un groupement sulfoxyde, un groupement sulfone, un groupement oxyde de phosphine, un groupement phosphonate obtenu par addition d'oxygène sur les atomes de soufre ou de phosphore peuvent conférer au polymère obtenu des propriétés améliorées en ce qui concerne l'adhésion, la brillance, la résistance à l'oxydation ou aux UV.

Les monomères et les prépolymères qui peuvent être polymérisés ou réticulés à l'aide des photoamorceurs de la présente invention sont ceux qui peuvent subir une polymérisation cationique.

Parmi les monomères, on peut citer les monomères qui comportent une fonction éther cyclique, une fonction thioéther cyclique ou une fonction amine cyclique les composés vinyliques, (plus particulièrement les éthers vinyliques), les oxazolines, les lactones et les lactames.

Parmi les monomères du type éther ou thioéther cyclique, on peut citer l'oxyde d'éthylène, l'oxyde de propylène, l'oxétane, l'épichlorhydrine, le tétrahydrofurane, l'oxyde de styrène, l'oxyde de cyclohexène, l'oxyde de vinylcyclohexène, le glycidol, l'oxyde de butylène, l'oxyde d'octylène, les éthers et les esters de glycidyle (par exemple le méthacrylate ou l'acrylate de glycidyle, le phényl glycidyl éther, le diglycidyléther de bisphénol A ou ses dérivés fluorés), les acétals cycliques ayant de 4 à 15 atomes de carbone (par exemple le dioxolane, le 1,3-dioxane, le 1,3-dioxépane).

Parmi les composés vinyliques, les éthers vinyliques constituent une famille très importante de monomères sensibles en polymérisation cationique. A titre d'exemple, on peut citer l'éthyl vinyl éther, le propyl vinyl éther, l'isobutyl vinyl éther, l'octadécyl vinyl éther, l'éthylèneglycol monovinyl éther, le diéthylèneglycol divinyl éther, le butanediol monovinyl éther, le butanediol divinyl éther, l'hexanediol divinyl éther, l'éthylèneglycol butyl vinyl éther, le triéthylèneglycol méthyl vinyl éther, le cyclohexanediméthano monovinyl éther, le cyclohexanediméthanol divinyl éther, le 2-éthylhexyl vinyl éther, le poly-THF-divinyl éther ayant une masse comprise entre 150 et 5000, le diéthylèneglycol monovinyl éther, le triméthylolpropane trivinyl éther, l'aminopropyl vinyl éther, le 2-diéthylaminoéthyl vinyl éther.

On peut également citer les éthers méthyl-vinyliques qui contiennent un ou plusieurs groupements CH₃CH=CH-O- et qui sont avantageusement obtenus par isomérisation des éthers allyliques correspondants en présence d'un catalyseur tel que (Φ₃P)₂RuCl₂.

Comme autres composés vinyliques qui peuvent être soumis à une polymérisation cationique en présence d'un composé polyionique de l'invention utilisé en tant que photoamorceur, on peut citer à titre d'exemple les 1,1-dialkyléthylènes (par exemple l'isobutène), les monomères aromatiques vinyliques (par exemple le styrène, les α-alkylstyrène, notamment l'α-méthylstyrène, le 4-vinylanisole, l'acénaphtène), les composés N-vinyliques (par exemple la N-vinylpyrolidone ou les N-vinyl sulfonamides).

Parmi les prépolymères, on peut citer les composés dans lesquels des groupements époxy sont portés par une chaîne aliphatique, une chaîne aromatique, ou une chaîne hétérocyclique, par exemple les éthers glycidiques du bisphénol A éthoxylés par 3 à 15 unités d'oxyde d'éthylène, les siloxanes possédant des groupements latéraux du type époxycyclohexène-éthyle obtenus par hydrosilylation des copolymères de di alkyl, d'alkylaryle ou de diaryl siloxane avec le méthyl hydrogénosiloxane en présence d'oxyde de vinylcyclohexène, les produits de condensation du type solgel obtenus à partir du triéthoxy ou du triméthoxy silapropylcyclohexène oxyde, les uréthanes incorporant les produits de réaction du butanediol monovinyléther et d'un alcool de fonctionnalité supérieure ou égale à 2 sur un di ou un tri isocyanate aliphatique ou aromatique.

Le procédé de polymérisation selon l'invention comprend une première étape au cours de laquelle on mélange au moins un monomère ou prépolymère capable de polymériser par voie cationique et au moins un composé ionique A⁺X⁻ ou (A⁺X⁻)ₚ de l'invention, et une deuxième étape au cours de laquelle on soumet le mélange obtenu à un rayonnement actinique ou un rayonnement β. De préférence, le mélange réactionnel est soumis au rayonnement après avoir été mis sous forme d'une couche mince ayant une épaisseur inférieure à 5 mm, de préférence sous forme d'un film mince ayant une épaisseur inférieure ou égale à 500 µm. La durée de la réaction dépend de l'épaisseur de l'échantillon et de la puissance de la source à la longueur d'onde λ active. Elle est définie par la vitesse de défilement devant la source, qui est comprise entre 300 m/min et 1 cm/min. Des couches de matériau final ayant une épaisseur supérieure à 5 mm peuvent être obtenues en répétant plusieurs fois l'opération consistant à épandre une couche et à la traiter par le rayonnement.

Généralement, la quantité de photoamorceur utilisé est comprise entre 0,01 et 15 % en poids par rapport au poids de monomère ou de prépolymère, de préférence entre 0,1 et 5 % en poids.

Un composé ionique A⁺X⁻ de la présente invention peut être utilisé comme photoamorceur en l'absence de solvant, notamment lorsque l'on souhaite polymériser des monomères liquides dans lesquels le sel est soluble ou aisément dispersable. Cette forme d'utilisation est particulièrement intéressante, car elle permet de supprimer les problèmes liés aux solvants (toxicité, inflammabilité).

Un composé ionique A⁺X⁻ de la présente invention peut également être utilisé en tant que photoamorceur sous forme d'une solution homogène dans un solvant inerte vis-à-vis de la polymérisation, prête à l'emploi et aisément dispersable, en particulier dans le cas où le milieu à polymériser ou à réticuler présente une viscosité élevée.

Comme exemple de solvant inerte, on peut citer les solvants volatils, tels que l'acétone, la méthyl-éthyl cétone et l'acétonitrile. Ces solvants serviront simplement à diluer les produits à polymériser ou à réticuler (pour les rendre moins visqueux, surtout lorsqu'il s'agit d'un prépolymère). Ils seront éliminés après la polymérisation ou la réticulation par séchage. On peut également citer les solvants non volatils. Un solvant non volatil sert également à diluer les produits que l'on veut polymériser ou réticuler, et à dissoudre le sel A⁺X⁻ de l'invention utilisé comme photoamorceur, mais il restera dans le matériau formé et il agit ainsi comme plastifiant. A titre d'exemple, on peut citer le carbonate de propylène, la γ-butyrolactone, les éther-esters des mono-, di-, tri- éthylène ou propylène glycols, les éther-alcools des mono-, di-, tri-éthylène ou propylène glycols, les plastifiants tels que les esters de l'acide phtalique ou de l'acide citrique.

Dans un autre mode de mise en oeuvre de l'invention, on utilise comme solvant ou diluant un composé réactif vis-à-vis de la polymérisation, qui est un composé de faible masse moléculaire et de faible viscosité qui va jouer à la fois le rôle de monomère polymérisable et le rôle de solvant ou de diluant pour des monomères plus visqueux ou des prépolymères utilisés conjointement. Après la réaction, ces monomères ayant servi de solvant font partie du réseau macromoléculaire finalement obtenu, leur intégration étant plus grande lorsqu'il s'agit de monomères bi-fonctionnels. Le matériau obtenu après irradiation ne contient plus de produits ayant un faible poids moléculaire et une tension de vapeur appréciable, ou susceptibles de contaminer les objets avec lesquels le polymère est en contact. A titre d'exemple, un solvant réactif peut être choisi parmi les mono et di éthers vinyliques des mono-, di-, tri-, tétra-éthylène et propylène glycols, la N-méthylpyrolidone, le 2-propényléther du carbonate de propylène commercialisé par exemple sous la dénomination PEPC par la société ISP, New Jersey, Etats-Unis.

Pour irradier le mélange réactionnel, le rayonnement peut être choisi parmi le rayonnement ultraviolet, le rayonnement visible, les rayons X, les rayons γ et le rayonnement β. Lorsque l'on utilise la lumière ultraviolette comme rayonnement actinique, il peut être avantageux d'ajouter aux photoamorceurs de l'invention des photosensibilisateurs destinés à permettre une photolyse efficace avec les longueurs d'ondes moins énergétiques que celles correspondant au maximum d'absorption du photoamorceur, telles que celles émises par les dispositifs industriels, (λ ≈ 300 nm pour les lampes à vapeur de mercure en particulier). De tels additifs sont connus, et à titre d'exemples non limitatifs, on peut citer l'anthracène, le diphényl-9,10-anthracène, le pérylène, la phénothiazine, le tétracène, la xanthone, la thioxanthone, l'acétophénone, la benzophénone, les 1,3,5-triaryl-2-pyrazolines et leurs dérivés, en particulier les dérivés de substitution sur les noyaux aromatiques par des radicaux alkyles, oxa- ou aza-alkyles permettant entre autre de changer la longueur d'onde d'absorption. L'isopropylthioxantone est un exemple de photosensibilisateur préféré lorsque l'on utilise un composé iodonium selon l'invention comme photoamorceur.

Parmi les différents types de rayonnement mentionnés, le rayonnement ultraviolet est particulière préféré. D'une part, il est plus commode d'emploi que les autres rayonnements mentionnés. D'autre part, les photoamorceurs sont en général directement sensibles aux rayons UV et les photosensibilisateurs sont d'autant plus efficaces que la différence d'énergie (δλ) est plus faible.

Les composés ioniques de l'invention peuvent aussi être mis en oeuvre en association avec des amorceurs de type radicalaire générés thermiquement ou par action d'une radiation actinique. Il est ainsi possible de polymériser ou de réticuler des mélanges de monomères ou de prépolymères contenant des fonctions dont les modes de polymérisation sont différents, par exemple des monomères ou des prépolymères polymérisant par voie radicalaire et des monomères ou des prépolymères polymérisant par voie cationique. Cette possibilité est particulièrement avantageuse pour créer des réseaux interpénétrés ayant des propriétés physiques différentes de celles qui seraient obtenues par simple mélange des polymères issus des monomères correspondants. Les éthers vinyliques ne sont pas ou sont peu actifs par amorçage radicalaire. Il est donc possible, dans un mélange réactionnel contenant un photoamorceur selon l'invention, un amorceur radicalaire, au moins un monomère du type éther vinylique et au moins un monomère comprenant des doubles liaisons non activées telles que celles des groupes allyliques, d'effectuer une polymérisation séparée de chaque type de monomère. Il est par contre connu que les monomères déficients en électrons, tels que les esters ou les amides de l'acide fumarique, de l'acide maléique, de l'acide acrylique ou méthacrylique, de l'acide itaconique, de l'acrylonitrile, du méthacrylonitrile, la maléimide et ses dérivés, forment en présence d'éthers vinyliques riches en électrons, des complexes de transfert de charge donnant des polymères alternés 1:1 par amorçage radicalaire. Un excès initial de monomères vinyliques par rapport à cette stoechiométrie permet de préserver des fonctions polymérisables par initiation cationique pure. Le déclenchement de l'activité d'un mélange d'amorceur radicalaire et d'amorceur cationique selon l'invention peut être fait simultanément pour les deux réactifs dans le cas par exemple d'insolation par un rayonnement actinique d'une longueur d'onde pour laquelle les photoamorceurs de l'invention et les amorceurs radicalaires choisis sont actifs, par exemple à λ = 250 nm. A titre d'exemple, on peut citer comme amorceurs les produits commerciaux suivants : Irgacure 184®, Irgacure 651®, Irgacure 261® Quantacure DMB®, Quantacure ITX®.

Il peut aussi être avantageux d'utiliser les deux modes de polymérisation d'une manière séquentielle, pour former dans un premier temps des prépolymères dont la mise en forme est aisée et dont le durcissement, l'adhésion, la solubilité ainsi que le degré de réticulation peuvent être modifiés par le déclenchement de l'activité de l'amorceur cationique. Par exemple, un mélange d'un amorceur radicalaire thermodissociable et d'un photoamorceur cationique selon l'invention permet de réaliser des polymérisations ou des réticulations séquentielles, d'abord sous l'action de la chaleur, puis sous l'action d'un rayonnement actinique. D'une manière similaire, si l'on choisit un amorceur radicalaire et un photoamorceur cationique selon l'invention, le premier étant photosensible à des longueurs d'ondes plus longues que celle déclenchant le photoamorceur selon l'invention, on obtient une réticulation en deux étapes contrôlables. Des amorceurs radicalaires peuvent être par exemple Irgacure® 651 permettant d'amorcer des polymérisations radicalaires à des longueurs d'onde de 365 nm.

L'invention a également pour objet l'utilisation composés ioniques A⁺X⁻ de l'invention pour les réactions d'amplification chimique de photoresists pour la microlithographie. Lors d'une telle utilisation, un film d'un matériau comprenant un polymère et un composé ionique de l'invention est soumis à une irradiation. L'irradiation provoque la formation de l'acide HX, qui catalyse la décomposition ou la transformation du polymère. Après décomposition ou transformation du polymère sur les parties du film qui ont été irradiées, les monomères formés ou le polymère transformé sont éliminés et il reste une image des parties non exposées. Pour cette application particulière, il est avantageux d'utiliser des composés polymères comprenant des unités vinyles portant un substituant ionique. Parmi ces composés, on peut citer les sels de polyiodonium répondant à la formule (II), les sels de polysulfonium répondant à la formule (VI), les polyacylsulfonium répondant à la formule (XI), les polydiazonium répondant à la formule (XV), les sels de complexes organométalliques répondant à la formule (VIII). Ces composés permettent d'obtenir après photolyse des produits qui ne sont pas volatils, et donc pas odorants lorsqu'il s'agit de sulfures. Parmi les composés de l'invention, on préfère tout particulièrement les polysulfonium qui sont particulièrement efficaces comme photoamorceur, les phénacylsulfonium et les polymères et copolymères de vinyl ferrocénium qui peuvent être obtenus facilement. Parmi les polymères qui peuvent ainsi être modifiés en présence d'un composé de l'invention, on peut citer notamment les polymères contenant des motifs ester ou des motifs aryléther d'alcool tertiaire, par exemple les poly(phtalaldéhydes), les polymères de bisphénol A et d'un diacide, le polytertiobutoxycarbonyl oxystyrène, le polytertiobutoxy-α-méthyl styrène, le polyditertiobutylfumarate-coallyltriméthylsilane et les polyacrylates d'un alcool tertiaire, en particulier le polyacrylate de tertiobutyle. D'autres polymères sont décrits dans J.V. Crivello et al, Chemistry of Materials 8, 376-381, (1996).

Les composés ioniques A⁺X⁻ de la présente invention, qui présentent une grande stabilité thermique, offrent de nombreux avantages par rapport aux sels connus de l'art antérieur.

La présente invention est décrite ci-après plus en détail, par les exemples suivants qui sont donnés à titre d'illustration.

### Exemple 1

A 100 ml d'une solution d'eau déionisée qui contient 15 g de bis-fluorosulfonimidure de potassium KN(SO₂F)₂ et qui a été maintenue à 0°C, on a ajouté 21 g (1,43 x 10⁻³ mole) de chlorure de diphényl-iodonium, (C₆H₅)₂ICl. Le mélange a été agité pendant 1 heure à l'abri de la lumière. Le précipité formé, correspondant au bis-fluorosulfonylimidure de diphényliodonium [N(SO₂F)₂]-[(C₆H₅)₂I]⁺, a été séparé par filtration, lavé à l'eau froide et séché sous vide.
Rendement : 91 % (27,8 g)
Analyse élémentaire: H : 2,2% ; C : 31,4% ; N : 3% ; F : 8,24% ; S : 14,1% ; I : 27,6%

### Exemple 2

0,16 g d'hydrure de lithium ont été mis en suspension dans 25 ml de THF anhydre. 1,53 ml de trifluoroéthanol ont été ajoutés goutte à goutte et sous agitation. Après la fin de la réaction et du dégagement d'hydrogène, on a obtenu le trifluoroéthoxyde de lithium. La solution a été filtrée et refroidie à 0°C. On a alors ajouté par portions, 2,03 g de bis-fluorosulfonylimidure de sodium, NaN(SO₂F)₂. Après une heure de réaction à 25°C, le précipité de fluorure de lithium a été séparé par centrifugation. Le solvant a été éliminé à l'aide d'un évaporateur rotatif et le produit résultant Na [(CF₃CH₂O)₂SO₂)₂N été dissous dans 50 ml d'eau. Après filtration, on a ajouté au filtrat 3,2 g de chlorure de diphényl-iodonium, (C₆H₅)₂ICl. Le précipité formé a été agité à 0°C pendant 2 heures à l'abri de la lumière puis séparé par filtration, lavage à l'eau froide et séchage sous vide. On a obtenu 7,2 g (rendement 88%) de [(CF₃CH₂O)₂SO₂)₂N]⁻ [(C₆H₅)₂I]⁺.

### Exemple 3

Un échantillon de toluènesulfonate de (4-octyloxyphényl)phényle iodonium (en abrégé : C8-O-iodonium) a été préparé selon la méthode de Crivello et al. (*J. Polym. Sci: Part A: Polymer Chemistry,* (1989), 27, 3951-3968) en faisant réagir l'hydroxytosyloxyiodobenzène sur l'octyloxybenzène dans un mélange acétonitrile / acide acétique. L'octyloxybenzène a été obtenu au préalable par réaction du phénate de sodium sur le bromooctane dans un mélange eau/toluène par catalyse à transfert de phase en présence de bromure de tétrabutylammonium. A 8 g de ce composé en solution dans 60 ml de méthyléthylcétone (MEK) on a ajouté 3,02 g de bis-fluorosulfonylimidure de potassium. Le mélange a été agité magnétiquement pendant une heure à température ambiante puis filtré pour éliminer le toluènesulfonate de potassium insoluble. La solution a été versée dans un flacon taré adaptable sur un évaporateur rotatif et le solvant a été évaporé. Les dernières traces de MEK ont été éliminées sous vide de pompe primaire. Le résidu constitué par le bis-fluorosulfonylimidure de (4-octyloxyphényl)phényle iodonium se présente sous forme d'une huile visqueuse (8,04 g, 98%). On y a ajouté un poids égal de dichlorométhane pour obtenir un mélange fluide qui peut être manipulé. Les tests de solubilité ont été faits dans des flacons tarés après addition d'une quantité donnée de mélange et évaporation de la fraction volatile (CH₂Cl₂). Les résultats des essais sont indiqués ci-dessous :

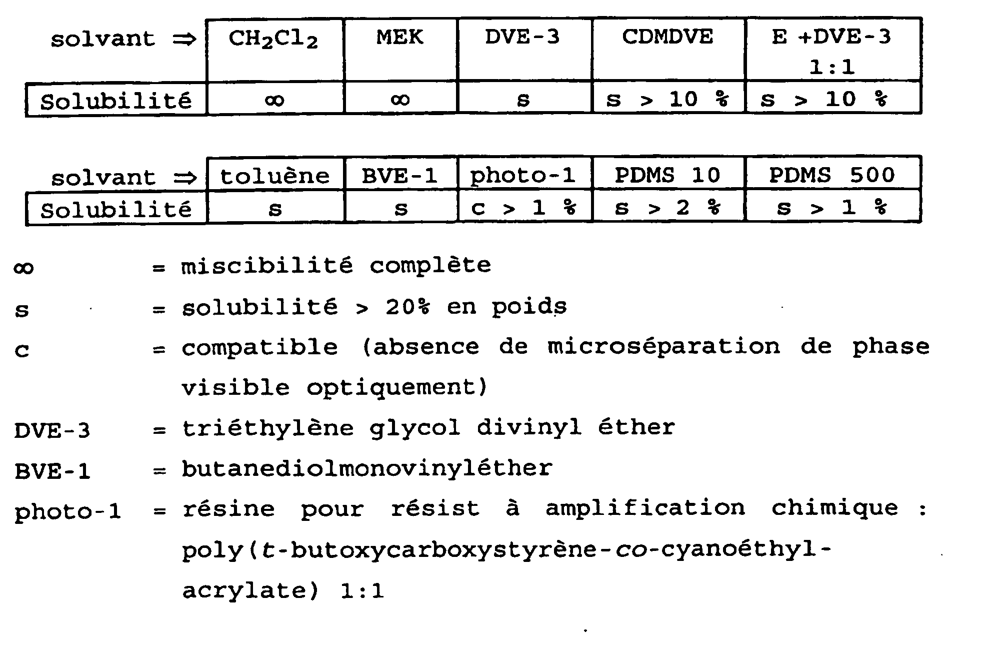

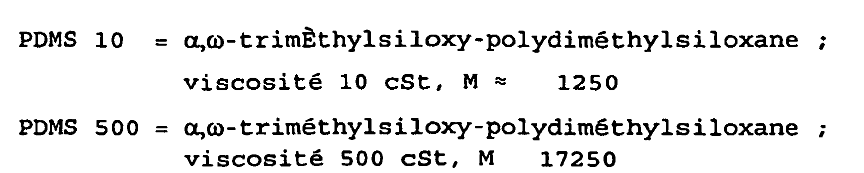

Cet exemple met en évidence les excellentes propriétés de solubilité de ces sels en particulier dans les solvants monomères et polymères de faible polarité et de caractère hydrophobe. Des résultats similaires sont obtenus avec les sels de bis-fluorosulfonimidure de (dodécyloxyphényl)-phényliodonium (C12-O-iodonium) et d'octadécyloxy phényl)phényliodonium (C18-O-iodonium) ayant des chaînes aliphatiques de respectivement 12 et 18 atomes de carbone.

### Exemple 4

On a fait réagir 19,3 g de bromure de 2-éthylhexylmagnésium préparé à partir du bromure de 2-éthylhexyle avec 15,7 g de bromobenzène en présence de 500 mg de catalyseur de bis[bis(diphénylphosphinoéthane]palladium(0). 19 g de 2-éthylhexyl-benzène ainsi obtenu ont été dissous dans 60 ml d'un mélange équivolumique d'acide et d'anhydride trifluoroacétique maintenu à 0°C sous agitation, auquel on a ajouté 3,5 g d'iodure de sodium et 4,2 g d'anhydride iodique. Le solvant a été éliminé à l'aide d'un évaporateur rotatif. Ensuite, le produit résiduel a été dissous dans 30 ml d'acétonitrile, puis versé dans 200 ml d'eau contenant 10 g d'acétate de sodium, 2 g de sulfite de sodium et 14 g de bis-fluorosulfonylimidure de potassium. La phase organique a été extraite à l'aide de dichlorométhane et lavée à l'eau. Le solvant a été éliminé à l'aide d'un évaporateur rotatif pour laisser un liquide huileux de bis-fluorosulfonylimidure de bis-[4-(2-éthylhexyl-)]phényl-iodonium.

Ce composé ionique présente des caractéristiques de solubilité dans les hydrocarbures et les huiles de silicones comparables à celles de composés à chaînes alkyles plus longues tels que le bis(dodécylbenzène) iodonium associé à l'anion PF₆⁻. Il a un caractère lipophile encore plus marqué que celui du composé de l'exemple 3. Il est particulièrement avantageux pour catalyser la réticulation des revêtements de silicone antiadhésifs et la polymérisation des monomères de faible polarité comme le cyclohexanediméthanoldivinyl-éther, le 2-éthylhexylglycidyl-éther ou le 2-éthylhexyl-vinyléther.

### Exemple 5

A 40 g d'allyloxybenzène dans 50 ml d'acide acétique on a ajouté goutte à goutte 78,4 g de phényliodosotoluènesulfonate dans 100 ml d'acide acétique, puis on a agité à température ordinaire pendant 2 heures. Le mélange réactionnel a ensuite été versé dans 1 l de dioxane, le précipité de toluènesulfonate d'(allyloxyphényl)-phényliodonium obtenu a été lavé à l'éther et séché. La transformation en bis-fluorosulfonylimidure a été effectuée en mélangeant 15 g de toluènesulfonate d'(allyloxyphényl) phényliodonium et 7 g de KN(SO₂F)₂ dans 150 ml d'eau à 0°C. Le sel formé a été extrait avec 2 x 50 ml de dichlorométhane, puis le solvant a été évaporé.

Le sel résiduel (constitué par le bis-fluorosulfonylimidure d'(allyloxyphényl)-phényliodonium) a été dilué par 60 ml de THF et introduit dans un ballon tricol équipé d'un réfrigérant, d'une agitation mécanique et d'une entrée de gaz neutre (Argon), ledit ballon contenant 8,5 g d'un copolymère de diméthylsiloxane et d' (hydrogéno)(méthyl)-siloxane (HMS 301 25% SiH, M_{w} 1900 commercialisé par Gelest Inc., Tullytown, PA, USA) en solution dans 60 ml de THF, ainsi que 90 mg d'acide chloroplatinique H₂PtCl₆. Le mélange a été chauffé au reflux pendant 2 heures. On a ensuite ajouté 1 g de 1-hexène et la réaction a été continuée pendant 1 heure. Un prélèvement a montré la disparition complète de bandes IR de la liaison SiH, ce qui confirme l'hydrosilylation entre les groupements allyles du bis-fluorosulfonylimidure d'(allyloxyphényl)-phényliodonium) et les groupes SiH du copolymère de diméthylsiloxane et d' (hydrogéno) (méthyl)-siloxane. Le polymère ainsi obtenu, qui contient des groupes ioniques pendants, est précipité dans le méthanol et purifié par trois opérations de dissolution (THF) / précipitation (méthanol puis éther). Il peut être représenté par la formule suivante :

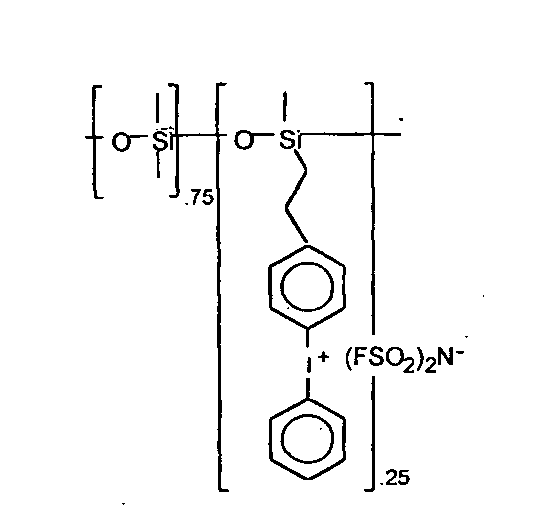

### Exemple 6

6,5 g de trifluorométhanesulfonamide CF₃SO₂NH₂ et 10,8 ml de pyridine dans 60 ml de dichlorométhane ont été refroidis à -15°C et on a ajouté goutte à goutte 3,6 ml de chlorure de sulfuryle dans 10 ml de dichlorométhane, puis 4,6 ml de 1,1,1,3,3,3-hexafluoro-2-propanol. Le mélange a été agité pendant 1 heure à -15°C, puis 4 heures à température ambiante (25°C). Le mélange réactionnel a été filtré et le solvant a été éliminé à l'aide d'un évaporateur rotatif ; le résidu solide obtenu a été dissous dans 50 ml d'eau contenant 5 g d'acétate de sodium, 18,45 g de bis(hexafluorophosphate de bis[4-(diphénylsulfonio)phényl]thioéther) finement pulvérisé (préparé au préalable selon la méthode d'Akhtar et al. (*Chem. Mat.* (1990), 2, 732 et K.T. Chang, US 4,197,174) ont été ajoutés, et le mélange a été agité à température de la pièce pendant 3 h dans un broyeur à rouleaux dans un flacon de polyéthylène rigide contenant des boulets d'oxyde de zirconium. La suspension obtenue a été filtrée et le solide résultant a été séché. On a ainsi obtenu 28 g (rendement : 78%) du composé de structure suivante :

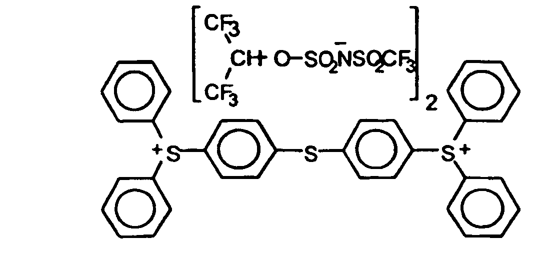

### Exemple 7

Le méthanesulfonate de bis(4-fluorophényl)-[4(4-benzoylphénylthio)phényl]sulfonium a été préparé selon la méthode de Abe et al. (US 5,534,557) par réaction de Friedel et Craft de l'acide benzoique sur le diphénylthioéther, et condensation dans le réactif d'Eaton (P₂O₅ / CH₃SO₃H) (*J. Org. Chem.* (1973), 38, 4071) de la manière suivante : 15 g de (4-benzoyl-diphénylthioéther, 12,4 g de 4,4'-difluorodiphénylsulfoxide et 20,6 g de pentoxyde de phosphore ont été dissous dans 206 g d'acide méthane sulfonique et le mélange réactionnel a été maintenu pendant 3 heures à 80°C.

La solution contenant le méthanesulfonate de bis(4-fluorophényl)-[4(4-benzoylphénylthio)phényl]sulfonium qui s'est formé au cours de la réaction a été versée dans 700 ml d'eau contenant 200 g d'acétate de sodium et 12 g de bis-fluorosulfonylimidure de potassium. Le précipité de bis-fluorosulfonylimidure de bis (4-fluorophényl)-[4(4-benzoylphénylthio)phényl]sulfonium qui s'est formé a été séparé par filtration et recristallisé dans le 2-propanol. Analyse élémentaire : H : 3.06% ; C : 53.8% ; N : 2.03% ; F : 11% ; S : 18.6%.

### Exemple 8

13,2 g de thiophénate de sodium et 19,04 g de 9,10-dibromoanthracène ont été dissous dans 100 ml de N-méthylpyrrolidone à 180°C. Le produit réactionnel (DPA) a été lavé à l'eau, puis recristallisé dans un mélange chloroforme / éthanol.

6 g de DPA, 4,2 g de bromobutane et 10 g de tris(fluorosulfonyl)méthylure de potassium ont été chauffés dans 40 ml de MEK dans un flacon étanche à 60°C pendant 24 heures, puis le mélange résultant a été filtré pour éliminer le bromure de potassium insoluble. Le solvant a été éliminé à l'aide un évaporateur rotatif. On a obtenu le sel dont la structure est représentée par :

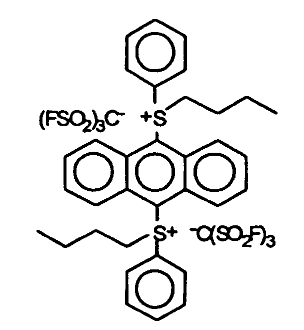

Ce sel est beaucoup plus actif, en fraction massique requise, ainsi qu'en cinétique de polymérisation, que les sels de sulfonium conventionnels. Contrairement à la méthode de préparation décrite dans Hacker et al. (US 4,760,013) pour le sel de SbF₆⁻, aucun sel d'argent n'est requis pour la préparation de ce composé grâce à la solubilité des sels dérivés des anions de la présente invention dans des solvants dans lesquels les halogénures alcalins sont parfaitement insolubles.

### Exemple 9

Un polythioéther a été préparé par réaction de 15 g de dimercaptohexane avec 18,67 g de 1,2-bis(2-chloroéthoxy)éthane dans 200 ml de N-méthylpyrrolidone à 150°C en présence de 10 g de carbonate de potassium. Le polymère a été précipité dans l'eau et purifié par plusieurs opérations de dissolution (THF) / précipitation (diéthyléther) et séparation par centrifugation dans un récipient unique. Le polymère se présente sous la forme d'une masse collante.

Dans un ballon tricol équipé d'un réfrigérant, d'une agitation mécanique et d'une entrée de gaz neutre (Argon), 7 g du polymère ont été dissous dans 120 ml de dichlorométhane (CH₂Cl₂) et on a ajouté goutte à goutte 4,8 g de 2-bromoacétophénone. Le mélange a été chauffé au reflux à 40°C. Un précipité est apparu rapidement. La réaction a été poursuivie 12 heures après l'apparition du précipité. Le bromure de polyphénacylsulfonium formé a été séparé par filtration et lavé avec du dichlorométhane et de l'éther.

5 g bromure de polyphénacylsulfonium) ont été dissous dans 60 ml d'eau, la solution a été filtrée et 2,5 g de sel de potassium de bis(fluorosulfonyl)imide K(FSO₂)₂N dans 25 ml d'eau ont été ajoutés sous agitation. Un précipité s'est formé immédiatement et l'agitation a été poursuivie pendant 1 h. Le polymère bisfluorosulfonylimidure de polyphénacylsulfonium a été séparé par filtration et séché (rendement quantitatif).

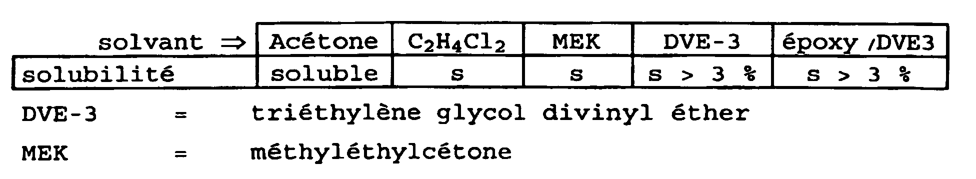

### Exemple 10

Les propriétés de solubilité de certains composés polyioniques de la présente invention ont été comparées aux propriétés d'un poly(iodonium) préparé selon Crivello & Lam (*J. Polym. Sci.; Polymer Chemistry,* (1979), 17, 3845-3858). Le polyiodonium selon Crivello et al a été obtenu par une trans-addition du chlorure de (4,4'-N-maleimido)diphényliodonium avec le 1,10-décanethiol dans le m-crésol en présence d'une base tertiaire, suivie d'une métathèse en présence de NaPF₆. Les composés de la présente invention ont été préparés de la même manière, mais en remplaçant pour la métathèse NaPF₆ par l'un des sels suivants : Na⁺(FSO₂)₂N⁻, Na⁺[(CF₃)₂CHOSO₂]₂N⁻, Na⁺(FSO₂)₃C⁻. Les résultats sont groupés dans le tableau suivant :

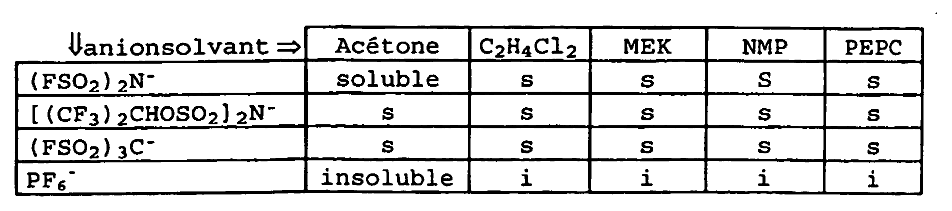

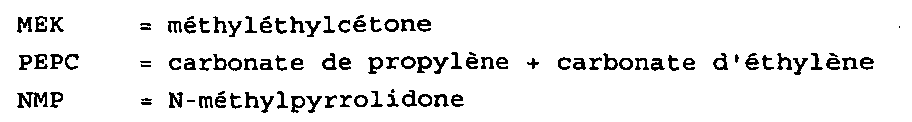

Des différences marquées de solubilité sont aussi observées avec les sels de polysulfonium linéaires décrits par Kuczinski (US 5,550,171) qui ne sont solubles que dans des solvants de paramètre de solubilité égal à 22 MPa^{1/2}, correspondant à la N-méthylpyrrolidone NMP et au carbonate de propylène. Les photoamorceurs obtenus en remplaçant l'anion MF₆⁻ (M = P, As, Sb) par ceux de l'invention tels que (FSO₂)₂N⁻ ou [(CF₃)₂CHOSO₂]₂N⁻ deviennent solubles dans des solvants communs et faciles d'emploi ayant des paramètres de solubilité inférieurs à 15 MPa^{1/2} tels que la méthyléthylcétone, l'acétone, l'acétonitrile.

### Exemple 11

10 g d'hexafluorophosphate de (cumène)cyclopentadiényle-fer (Irgacure 261®, Ciba-Geigy, Suisse) (noté [[Fe]]⁺PF₆⁻) ont été mis en solution dans 50 ml de dichloroéthane. A cette solution ont été ajoutés 5,7 g de bis-fluorosulfonylimidure de potassium en solution dans 15 ml de nitrométhane. Le mélange a été agité 1 heure et le précipité d'hexafluorophosphate de potassium KPF₆ a été séparé par filtration. Le solvant a été éliminé à l'aide d'un évaporateur rotatif pour laisser une poudre orange constituée par le bis-fluorosulfonylimidure de (cumène)-cyclopentadiényle-fer, très soluble dans les solvants usuels et les monomères vinyliques.

Les résultats des essais de solubilité sont donnés ci-après.

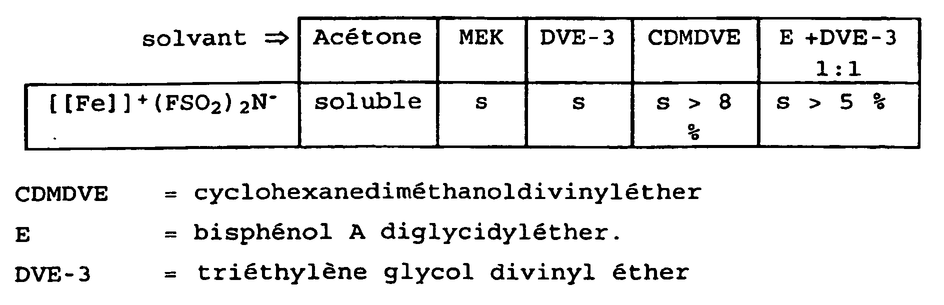

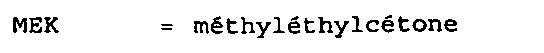

Selon le même procédé, on a fait réagir 10 g d'Irgacure® 261 et 7 g de (fluorosulfonyl) (trifluorométhanesulfonyl)imidure de potassium K(FSO₂)NSO₂CF₃ pour obtenir le (fluorosulfonyl)(trifluorométhanesulfonyl)imidure de (cumène)cyclopentadiényle-fer [[Fe]]⁺[(FSO₂)NSO₂CF₃]⁻ d'une manière quantitative.

### Exemple 12

A 10 g de butylferrocène (Aldrich, Milwaukee USA) en solution dans 50 ml d'acétonitrile maintenus à 0°C on a ajouté 1 ml de brome dilué dans 5 ml de d'acétonitrile. A la solution bleue obtenue, on a ajouté 9,5 g bis-fluorosulfonylimidure de potassium. Le mélange a été agité 1 heure et le précipité de bromure de potassium a été séparé par filtration. Le solvant a été éliminé à l'aide d'un évaporateur rotatif. On obtient une huile bleu foncé qui recristallise par trituration avec de l'éther. Le bis-flurosulfonylimidure de butylferricinium obtenu sous forme de cristaux, a été lavé à l'éther et conservé à l'abri de la lumière.

Analyse élémentaire: H 4.7; C 43,8; N 3.65; F 14.8; S 16.8.

### Exemple 13

A 5 g de 1,2-diferrocényléthane (commercialisé par Aldrich C°, Milwaukee, USA) en solution dans 50 ml de toluène on a ajouté 3,4 g de [bis(trifluoroacétoxy)iodo]-benzène. Un précipité bleu s'est formé immédiatement. Il a été séparé, lavé à l'éther et séché. 5 g du solide obtenu ont été mis en solution dans 25 ml d'eau et on y a ajouté 3,6 g de bis(fluorosulfonyl)imidure de potassium. Le précipité du dimère du ferrocène sous forme de sel de l'anion imidure a été obtenu avec un rendement quantitatif sous forme d'une poudre cristalline bleue.

### Exemple 14

A 6 g d'un copolymère de vinylferrocène (commercialisé par Aldrich C°, Milwaukee, USA) et de méthacrylate de butyle contenant 42% de motifs organométalliques obtenu par polymérisation radicalaire induite par l'azobis(butyronitrile) en solution dans le toluène on a ajouté 3,3 g de [bis(trifluoroacétoxy)iodo]benzène (commercialisé par Aldrich). Un précipité bleu s'est formé immédiatement et a été séparé, lavé à l'éther pour éliminer l'excès d'oxydant et séché. 5 g de ce composé poly(ionique) de ferricinium associé à l'anion trifluoroacétate ont été mis en suspension dans 25 ml d'eau à laquelle ont été ajoutés 2,2 g de bis(fluorosulfonyl)imidure de potassium. Le précipité du composé polyionique de l'anion imidure du polyferricinium a été obtenu avec un rendement quantitatif sous forme d'une poudre amorphe bleue soluble dans la plupart des solvants usuels.

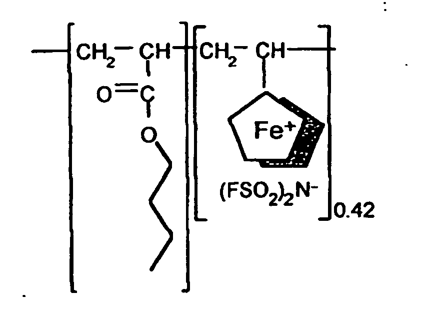

### Exemple 15

Un oligomère de diazonium a été préparé selon la méthode décrite dans U.S. 2,714,066, par condensation du chlorozincate de 4-diazodiphénylamine avec le formaldéhyde. 25 g de ce composé poly(ionique) ont été dissous dans 500 ml d'eau maintenue à 0°C et contenant 50 g d'acétate de sodium et 28 g de di-sel de sodium de l'acide éthylènediaminetétraacétique (EDTA). On a ajouté alors 17 g de sel de potassium de la bis(fluorosulfonyl)imide en solution dans 50 ml d'eau. Le composé poly(ionique) a été séparé par filtration et séché (rendement quantitatif), et conservé à 0°C à l'abri de la lumière. Ce composé est très soluble dans les solvants organiques usuels moyennement polaires tels que la MEK, et soluble dans les monomères de type DVE-3 ou PEPC et les éthers glycidiques.

### Exemple 16

### Photoresist négatif

2g de poly(4-hydroxystyrène)-co-styrène (8:2) (commercialisé par Shinetsu, Japon) en solution dans 20 ml diméthylformamide ont été ajoutés à 9,7 ml d'une solution 1M d'hydroxyde de potassium dans le méthanol contenant 1,3 g de chloroéthylvinyléther. La solution a été chauffée à 80°C pendant 1 heure et le mélange réactionnel a été versé dans 100 ml d'eau où le poly(4-vinyloxyéthyl)-styrène-co-styrène formé a précipité. Le polymère a été purifié par plusieurs opérations de dissolution précipitation dans l'acétone (solvant) / eau (précipitant) et acétone (solvant) / éther (précipitant).

1 g de poly(4-vinyloxyéthyl)-styrène-co-styrène et 20 mg du polymère de l'exemple 5 dans 10 ml de MEK ont été déposés à la tournette sur un substrat de silicium de manière à former un film de 0,5 µm d'épaisseur. Le film a été soumis à une exposition de 1 MJ/cm² obtenue par un laser KrF à travers un masque interférentiel. Le développement a été effectué par le THF. La résolution obtenue, observée par microscope électronique (SEM) est de l'ordre de l'épaisseur du film, soit 0,5 µm. Ce photorésist ne contient aucun élément métallique susceptible de contaminer le silicium.

### Exemple 17

### Photorésist positif

1g de poly(4-t-butoxycarboxystyrène) dans le dichloroéthane et 60 mg du polymère de l'exemple 9 ont été déposés à la tournette sur un substrat de silicium de manière à former un film de 0,5 µm d'épaisseur. Le film a été soumis à une exposition de 1 MJ/cm² obtenue par un laser KrF à travers un masque interférentiel. Le développement a été effectué par une solution à 4% d'hydroxyde de tétraméthyl ammonium dans l'eau. La résolution obtenue, observée par microscope électronique (SEM) est de l'ordre de l'épaisseur du film, soit 0,5 µm. Ce photorésist ne contient aucun élément métallique susceptible de contaminer le silicium.

### Exemple 18

Les propriétés de photo-amorçage des produits de l'invention sont illustrées par les résultats rassemblés dans le tableau suivant. Les composés polyioniques des exemples précédents ont été utilisés à raison de 1% en poids dans différents monomères et irradiés par un rayonnement U.V. à 254 nm avec une puissance de 1900 mW/cm² pendant une durée de 5 secondes, suivie d'une période de 10 minutes permettant la propagation des espèces générées dans le milieu (postcure).

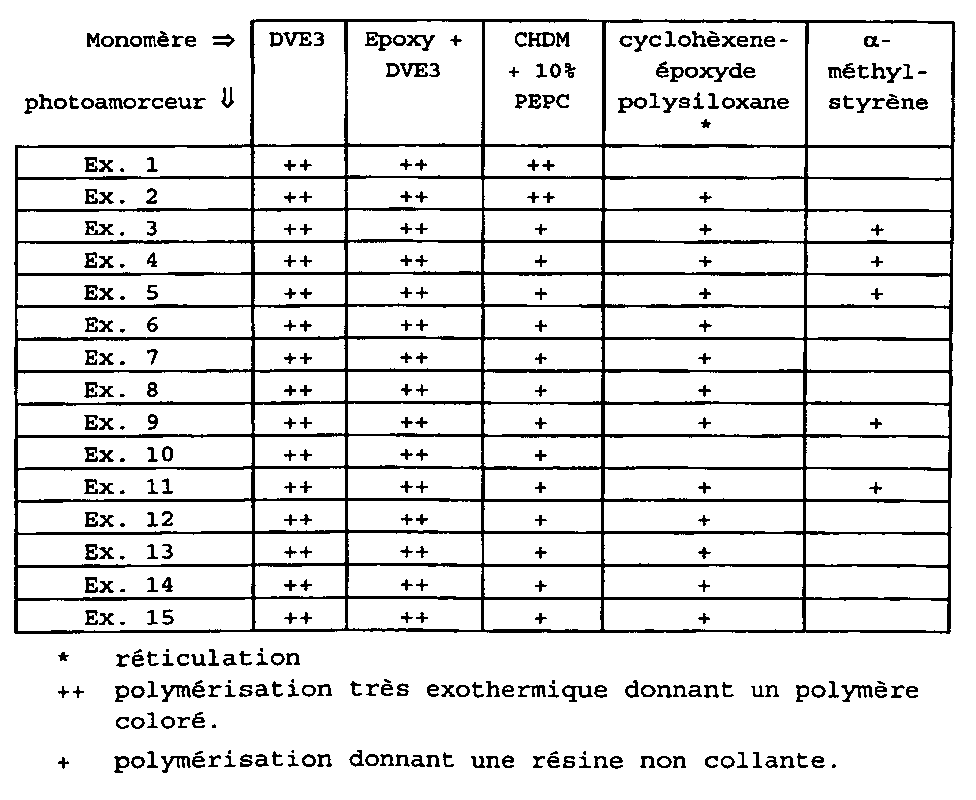

## Revendications

1. Composé ionique comprenant au moins un groupement A⁺X⁻, **caractérisé en ce que** :
- A⁺ est un groupe cationique choisi dans le groupe constitué par les groupements biaryliodonium, les groupements arylsulfonium, les groupements arylacylsulfonium, les groupements diazonium et les cations organométalliques comprenant un métal de transition complexé par au moins un cycle insaturé comprenant de 4 à 12 atomes de carbone, ledit groupe cationique faisant éventuellement partie d'une chaîne polymère ;
- X⁻ est un anion imidure [FSO₂NSO₂R'_{F}]⁻ ou [R_{F}CH₂OSO₂NSO₂R'_{F}]⁻ ou [(R_{F})₂CHOSO₂NSO₂R'_{F}]⁻, ou un anion méthylure [FSO₂C(Q)SO₂R'_{F}]⁻ ou [R_{F}CH₂OSO₂C(Q)SO₂R'_{F}]⁻ ou [(R_{F})₂CHOSO₂C(Q)SO₂R'_{F}]⁻ dans lesquels :
1) Q représente :
- H-, Cl-, F-, Br- ou CN- ;
- un radical alkyle ayant de 1 à 30 atomes de carbone ;
- un radical aryle ou alkylaryle ou arylalkyle ayant de 6 à 30 atomes de carbone ;
- un groupe R"_{F}-, un groupe R"_{F}SO₂-, un groupe R"_{F}CH₂O-SO₂- ou un groupe (R"_{F})₂CHO-SO₂- ;
2) R_{F} et R'_{F}, ainsi que R"_{F} le cas échéant lorsque X⁻ est un anion méthylure, sont choisis indépendamment l'un de l'autre dans le groupe constitué par le fluor, par les groupements perhaloalkyles ayant de 1 à 30 atomes de carbone, par les groupements (perhaloalkyl)alcoxy ayant de 2 à 30 atomes de carbone, par les groupements cycloaliphatiques perhalogénés ayant de 3 à 30 atomes de carbone contenant éventuellement des hétéroatomes choisis parmi O et N et/ou portant éventuellement au moins un chaînon perhaloalkyle, et par les groupements aryles perhalogénés ayant de 6 à 30 atomes de carbone; ou bien
3) R_{F} et R'_{F} forment ensemble un radical divalent choisi parmi les radicaux alkylènes linéaires perfluorés ayant de 2 à 8 atomes de carbone ; ou bien
4) lorsque X⁻ est un anion méthylure, R'_{F} et R"_{F} d'une part, ou R_{F} et R"_{F} d'autre part forment ensemble un radical divalent choisi parmi les radicaux alkylènes linéaires perfluorés ayant de 2 à 8 atomes de carbone.

2. Composé selon la revendication 1, **caractérisé en ce que** l'anion est un anion sulfonimidure répondant à l'une des formules suivantes [FSO₂NSO₂R'_{F}]⁻, [CF₃CH₂OSO₂NSO₂R'_{F}]⁻ et [(CF₃)₂CHOSO₂NSO₂R'_{F}]⁻, ou un anion sulfonylméthylure répondant à l'une des formules [FSO₂C(Q)SO₂R'_{F}]⁻, [CF₃CH₂OSO₂C(Q) SO₂R'_{F}]⁻ ou [(CF₃)₂CHOSO₂-C(Q)SO₂R'_{F}]⁻, dans lesquelles R'_{F} est F, CF₃CH₂O-, (CF₃)₂CH-O- ou un groupement perfluoroalkyle ayant de 1 à 10 atomes de carbone et Q est choisi dans le groupe constitué par l'hydrogène, FSO₂-, CF₃CH₂O-SO₂-, (CF₃)₂CH-O-SO₂-, les groupements alkyles, aryles, alkylaryles ou arylalkyles ayant au plus 30 atomes de carbone, les groupements perfluoroalkylsulfonyles ayant de 1 à 8 atomes de carbone, et les groupements perfluoroalkyles ayant de 1 à 12 atomes de carbone ; ou bien Q et R'_{F} forment ensemble un groupement divalent alkylène linéaire perfluoré ayant de 1 à 8 atomes de carbone.

3. Composé selon la revendication 1, **caractérisé en ce que** l'anion est choisi dans le groupe constitué par (FSO₂)₂N⁻, (FSO₂)₃C⁻, (FSO₂)₂CH⁻, (CF₃CH₂OSO₂)₂N⁻, [(CF₃)₂CHOSO₂]₂N⁻, (CF₃CH₂OSO₂)₂CH⁻, [(CF₃)₂CHOSO₂]₂CH⁻, [(CF₃)₂CHOSO₂]₃C⁻, [(CF₃)₂CHOSO₂]₃C⁻, [FSO₂NSO₂CF₃]⁻, [FSO₂NSO₂C₂F₅]⁻, [(CF₃)₂CHO-SO₂NSO₂CF₃]⁻, [(CF₃)₂CHOSO₂NSO₂CF₃]⁻, [(CF₃)₂CHOSO₂NSO₂C₂F₅]⁻ et [(CF₃CH₂OSO₂NSO₂C₂F₅]⁻.

4. Composé selon la revendication 1, **caractérisé en ce que** le cation A⁺ répond à la formule 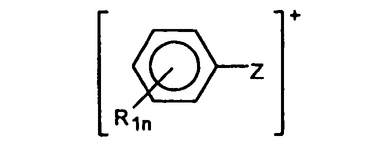 dans laquelle Z est choisi dans le groupe constitué par :
- les groupements aryliodonium 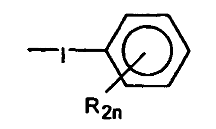
- les groupements sulfonium 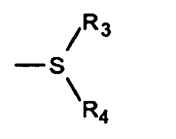
- les groupements acylsulfonium 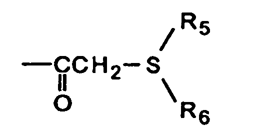
- les groupements diazonium
N≡N-R₈
- les groupements organométalliques 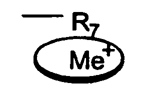 et dans laquelle R₁ₙ représente de 1 à 4, de préférence 1 à 2 groupements identiques ou différents liés à l'un quelconque des atomes de carbone libres du groupe aryle, R₂ₙ représente de 1 à 4, de préférence de 1 à 2 groupements identiques ou différents liés à l'un quelconque des atomes de carbone libres du groupe aryle, et les groupements R₁ₙ, R₂ₙ, et R₃ à R₈ sont choisis indépendamment les uns des autres parmi :
• les radicaux alkyles ou arylalkyles linéaires ou ramifiés ayant de 1 à 30 atomes de carbone ;
• les radicaux alkényles ayant de 1 à 30 atomes de carbone ;
• les radicaux aryles ou alkylaryles ayant de 6 à 30 atomes de carbone, incluant ceux qui ont des noyaux condensés ;
• les radicaux ayant de 1 à 30 atomes de carbone et choisis dans le groupe constitué par les oxaalkyles, les azaalkyles, les thiaalkyles, les phosphaalkyles, les oxaalkylènes, les azaalkylènes, les thiaalkylènes, les phosphaalkylènes,
• les radicaux ayant de 1 à 30 atomes de carbone et incluant un groupement sulfoxyde, un groupement sulfone, un groupement oxyde de phosphine, un groupement phosphonate, tous ces radicaux étant obtenus par addition d'oxygène sur les atomes de soufre ou de phosphore ;
• les radicaux hétérocycliques aromatiques ou alicycliques comprenant au moins un hétéroatome choisi dans le groupe constitué par O, N, S et P ;
• -NO, -CN, -OH, -Cl, -Br, -I, -F ;
• ou bien deux substituants choisis parmi les R₁ₙ et/ou deux substituants choisis parmi les R₂ₙ, ou les substituants R₃ et R₄, ou les substituants R₅ et R₆ forment ensemble un radical divalent qui forme un cycle avec le groupement qui les porte, ledit radical divalent étant choisi dans le groupe constitué par les radicaux alkylènes linéaires ayant de 1 à 18 atomes de carbone, les biradicaux benzo portant éventuellement au moins un substituant choisi de préférence dans le groupe constitué par les radicaux alkyles, oxaalkyles ou alcényles ayant de 1 à 10 atomes de carbone, les groupements oxaalkylènes répondant à la formule -R'-(OCH₂CH₂)_{q}-OR'- ou -R'-[OCH(CH₃)CH₂]_{q}-OR'- dans lesquels R' est H ou un radical alkylène linéaire ayant de 1 à 18 atomes de carbone et 1≤q≤22.

5. Composé selon la revendication 4, **caractérisé en ce que** les substituants R₁ₙ, R₂ₙ, et R₃ à R₈ sont choisis indépendamment les uns des autres dans le groupe constitué par les radicaux alkyles linéaires ayant de 1 à 18 atomes de carbone, le 2-éthylhexyle, le phényle, les oxaalkyles répondant à la formule R-(OCH₂CH₂)_{y}- ou R-[OCH(CH₃)CH₂]_{y}- dans lesquels R est H ou un radical alkyle linéaire ayant de 1 à 18 atomes de carbone et 1≤y≤22.

6. Procédé de polymérisation ou de réticulation de monomères ou de prépolymères capables de réagir par voie cationique, **caractérisé en ce que** l'on utilise un composé selon la revendication 1 comme photoamorceur source d'acide catalysant la réaction.

7. Procédé selon la revendication **6, caractérisé en ce que** les monomères sont choisis dans le groupe constitué par les composés qui comportent une fonction éther cyclique, une fonction thioéther cyclique ou une fonction amine cyclique, les composés vinyliques, les éthers vinyliques, les oxazolines, les lactones et les lactames.

8. Procédé selon la revendication **6, caractérisé en ce que** le prépolymère est choisi dans le groupe constitué par les composés dans lesquels des groupements époxy sont portés par une chaîne aliphatique, une chaîne aromatique, ou une chaîne hétérocyclique.

9. Procédé selon la revendication **6, caractérisé en ce qu'**il consiste à mélanger le photoamorceur avec au moins un monomère ou prépolymère capable de polymériser par voie cationique, et à soumettre le mélange obtenu à un rayonnement actinique ou un rayonnement β.

10. Procédé selon la revendication **9**, **caractérisé en ce que** le mélange réactionnel est soumis au rayonnement après avoir été mis sous forme d'une couche mince.

11. Procédé selon la revendication **6, caractérisé en ce que** la quantité de photoamorceur utilisé est comprise entre 0,01 et 15 % en poids par rapport au poids de monomère ou de prépolymère.

12. Procédé selon la revendication **6, caractérisé en ce que** le photoamorceur est utilisé sous forme d'une solution dans un solvant inerte vis à vis de la réaction de polymérisation.

13. Procédé selon la revendication **12**, **caractérisé en ce que** le solvant inerte est choisi dans le groupe constitué par l'acétone, la méthyl-éthyl cétone, l'acétonitrile, le carbonate de propylène, la γ-butyrolactone, les éther-esters des mono-, di-, tri-éthylène ou propylène glycols, les éther-alcools des mono-, di-, tri- éthylène ou propylène glycols, les esters de l'acide phtalique ou de l'acide citrique.

14. Procédé selon la revendication **6**, **caractérisé en ce que** la réaction est effectuée en présence d'un solvant ou d'un diluant constitué par un composé réactif vis à vis de la polymérisation.

15. Procédé selon la revendication **14, caractérisé en ce que** le composé réactif est choisi dans le groupe constitué par les mono et di éthers vinyliques des mono-, di-, tri-, tétra- éthylène ou propylène glycols, le trivinyl éther triméthylolpropane et le divinyléther du diméthanol-cyclohexane, la N-vinylpyrolidone, le 2-propényléther du carbonate de propylène.

16. Procédé selon la revendication **6, caractérisé en ce que** l'on ajoute un photosensibilisateur au milieu réactionnel.

17. Procédé selon la revendication **16, caractérisé en ce que** le photosensibilisateur est choisi dans le groupe constitué par l'anthracène, le diphényl-9,10-anthracène, le pérylène, la phénothiazine, le tétracène, la xanthone, la thioxanthone, l'isopropylthioxantone, l'acétophénone, la benzophénone, les 1,3,5-triaryl-2-pyrazolines et leurs dérivés, en particulier les dérivés de substitution sur les noyaux aromatiques par des radicaux alkyles, oxa- ou aza-alkyles.

18. Procédé selon la revendication **6**, **caractérisé en ce que** le mélange réactionnel contient en outre au moins un monomère ou prépolymère capable de polymériser par voie radicalaire et un composé capable de libérer un amorceur de polymérisation radicalaire sous l'effet du rayonnement actinique ou du rayonnement β ou sous l'action de la chaleur.

19. Procédé de modification des propriétés de solubilité d'un polymère possédant des groupements sensibles aux acides, **caractérisé en ce qu'**il consiste à soumettre ledit polymère à un rayonnement actinique ou un rayonnement β, en présence d'un composé selon la revendication 1.

20. Procédé selon la revendication 19, **caractérisé en ce que** le polymère contient des motifs ester ou des motifs aryléther d'alcool tertiaire.

21. Procédé selon la revendication 20, **caractérisé en ce que** le polymère est choisi dans le groupe constitué par les polyacrylates de tertiobutyle, les polyitaconates de tertiobutyle, le poly(tertiobutoxycarbonyloxystyrène), le poly(tertiobutyxostyrène).

22. Procédé selon la revendication 19, **caractérisé en ce qu'**il est mis en oeuvre pour l'amplification chimique de photorésists.

## Patentansprüche

1. Ionische Verbindung, umfassend zumindest eine Gruppierung A⁺X⁻, **dadurch gekennzeichnet, dass**:
- A⁺ eine kationische Gruppe, ausgewählt aus der aus Biaryliodonium-Gruppierungen, Arylsulfonium-Gruppierungen, Arylacylsulfonium-Gruppierungen, Diazonium-Gruppierungen und Organometallkationen, die ein Übergangsmetall umfassen, das mit zumindest einem ungesättigten Ring, der 4 bis 12 Kohlenstoffatome umfasst, komplexiert ist, worin die kationische Gruppe gegebenenfalls Teil einer Polymerkette ist, bestehenden Gruppe, ist;
- X⁻ ein Imidanion [FSO₂NSO₂R'_{F}]⁻ oder [R_{F}CH₂OSO₂NSO₂R'_{F}]⁻ oder [(R_{F})₂CHO-SO₂NSO₂R'_{F}]⁻ oder ein Methylanion [FSO₂C(Q)SO₂R'_{F}]⁻ oder [R_{F}CH₂OSO₂C(Q)-SO₂R'_{F}]⁻ oder [(R_{F})₂CHOSO₂C(Q)SO₂R'_{F}]⁻ ist, worin:
1) Q für:
- H-, Cl-, F-, Br- oder CN-;
- einen Alkylrest mit 1 bis 30 Kohlenstoffatomen;
- einen Aryl-, Alkylaryl- oder Arylalkylrest mit 6 bis 30 Kohlenstoffatomen;
- eine R"_{F}- Gruppe, eine R"_{F}SO₂-Gruppe, eine R"_{F}CH₂O-SO₂-Gruppe oder eine (R"_{F})₂CHO-SO₂-Gruppe steht;
2) R_{F} und R'_{F} sowie R"_{F}, wenn X⁻ ein Methylanion ist, unabhängig voneinander aus der aus Fluor, perhalogenierten Alkyl-Gruppierungen mit 1 bis 30 Kohlenstoffatomen, (Perhalogenalkyl)alkoxy-Gruppierungen mit 2 bis 30 Kohlenstoffatomen, perhalogenierten cycloaliphatischen Gruppierungen mit 3 bis 30 Kohlenstoffatomen, die gegebenenfalls Heteroatome, ausgewählt aus O und N, enthalten und/oder gegebenenfalls zumindest ein Perhalogenalkyl-Kettenglied tragen, und perhalogenierten Arylgruppierungen mit 6 bis 30 Kohlenstoffatomen bestehenden Gruppe ausgewählt sind; oder
3) R_{F} und R'_{F} zusammen einen zweiwertigen Rest, ausgewählt aus perfluorierten, unverzweigten Alkylenresten mit 2 bis 8 Kohlenstoffatomen, bilden; oder
4) wenn X⁻ ein Methylanion ist, R'_{F} und R"_{F} einerseits oder R_{F} und R"_{F} andererseits zusammen einen zweiwertigen Rest, ausgewählt aus perfluorierten unverzweigten Alkylenresten mit 2 bis 8 Kohlenstoffatomen, bilden.

2. Verbindung nach Anspruch 1, **dadurch gekennzeichnet, dass** das Anion ein Sulfonimidanion, für das eine der Formeln [FSO₂NSO₂R'_{F}]⁻, [CF₃CH₂OSO₂NSO₂R'_{F}]⁻ und [(CF₃)₂CHOSO₂NSO₂R'_{F}]⁻ gilt, oder ein Sulfonylmethylanion ist, für das eine der Formeln [FSO₂C(Q)SO₂R'_{F}]⁻, [CF₃CH₂OSO₂C(Q)SO₂R'_{F}]⁻ oder [(CF₃)₂CHOSO₂-C(Q)-SO₂R'_{F}]⁻ gilt, worin R'_{F} F, CF₃CH₂O-, (CF₃)₂CH-O- oder eine Perfluoralkylgruppierung mit 1 bis 10 Kohlenstoffatomen ist und Q aus der aus Wasserstoff, FSO₂-, CF₃CH₂O-SO₂-, (CF₃)₂CH-O-SO₂-, Alkyl-, Aryl-, Alkylaryl- oder Arylalkylgruppierungen mit höchstens 30 Kohlenstoffatomen, Perfluoralkylsulfonylgruppierungen mit 1 bis 8 Kohlenstoffatomen und Perfluoralkylgruppierungen mit 1 bis 12 Kohlenstoffatomen bestehenden Gruppe ausgewählt ist; oder Q und R'_{F} zusammen eine perfluorierte, unverzweigte, zweiwertige Alkylengruppierung mit 1 bis 8 Kohlenstoffatomen bilden.

3. Verbindung nach Anspruch 1, **dadurch gekennzeichnet, dass** das Anion aus der aus (FSO₂)₂N⁻, (FSO₂)₃C⁻, (FSO₂)₂CH⁻, (CF₃CH₂OSO₂)₂N⁻, [(CF₃)₂CHOSO₂]₂N⁻, (CF₃CH₂OSO₂)₂CH⁻, [(CF₃)₂CHOSO₂]₂CH⁻, [(CF₃)₂CHOSO₂]₃C⁻, [CF₃)₂CHOSO₂]₃C⁻, [FSO₂NSO₂CF₃]⁻, [FSO₂NSO₂C₂F₅]⁻, [(CF₃)₂CHO-SO₂NSO₂CF₃]⁻, [(CF₃)₂CHOSO₂N-SO₂CF₃]⁻, [(CF₃)₂CHOSO₂NSO₂C₂F₅]⁻ und [(CF₃CH₂OSO₂NSO₂C₂F₅]⁻ bestehenden Gruppe ausgewählt ist.

4. Verbindung nach Anspruch 1, **dadurch gekennzeichnet, dass** für das Kation A⁺ die Formel 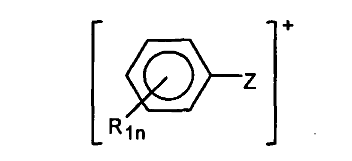 gilt, worin Z aus der aus
- Aryliodonium-Gruppierungen 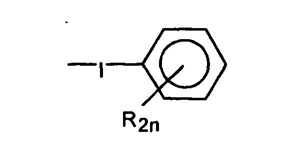
- Sulfonium-Gruppierungen 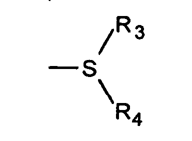
- Acylsulfonium-Gruppierungen 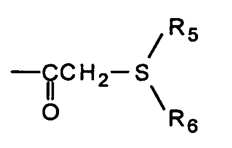
- Diazonium-Gruppierungen
N≡N-R₈
- organometallischen Gruppierungen 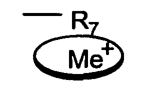 bestehenden Gruppe ausgewählt ist und worin R₁ₙ für 1 bis 4, vorzugsweise für 1 bis 2, gleiche oder unterschiedliche Gruppierungen steht, die an ein beliebiges der freien Kohlenstoffatome der Arylgruppe gebunden sind, R₂ₙ für 1 bis 4, vorzugsweise für 1 bis 2, gleiche oder unterschiedliche Gruppierungen steht, die an ein beliebiges der freien Kohlenstoffatome der Arylgruppe gebunden sind, und die Gruppierungen R₁ₙ, R₂ₙ und R₃ bis R₈ unabhängig voneinander aus:
- unverzweigten oder verzweigten Alkyl- oder Arylalkylresten mit 1 bis 30 Kohlenstoffatomen;
- Alkenylresten mit 1 bis 30 Kohlenstoffatomen;
- Aryl- oder Alkylarylresten mit 6 bis 30 Kohlenstoffatomen, einschließlich jenen mit kondensierten Ringen;
- Resten mit 1 bis 30 Kohlenstoffatomen, ausgewählt aus der aus Oxaalkylen, Azaalkylen, Thiaalkylen, Phosphaalkylen, Oxaalkylenen, Azaalkylenen, Thiaalkylenen und Phosphaalkylenen bestehenden Gruppe;
- Resten mit 1 bis 30 Kohlenstoffatomen, einschließlich einer Sulfoxidgruppe, einer Sulfongruppe, einer Phosphinoxidgruppe, einer Phosphonatgruppe, wobei alle diese Reste durch Addition von Sauerstoff an die Schwefelatome oder Phosphoratome erhalten werden;
- aromatischen heterozyklischen oder alizyklischen Resten, die zumindest ein aus der aus O, N, S und P bestehenden Gruppe ausgewähltes Heteroatom umfassen;
- -NO, -CN, -OH, -Cl, -Br, -I, -F;
bestehenden Gruppe ausgewählt sind;
- oder zwei Substituenten, ausgewählt aus den R₁ₙ, und/oder zwei Substituenten, ausgewählt aus den R₂ₙ, oder die Substituenten R₃ und R₄ oder die Substituenten R₅ und R₆ zusammen einen zweiwertigen Rest bilden, der mit der Gruppierung, die sie trägt, einen Ring bildet, worin der zweiwertige Rest aus der aus unverzweigten Alkylenresten mit 1 bis 18 Kohlenstoffatomen, Benzo-Biradikalen, die gegebenenfalls zumindest einen Substituenten, vorzugsweise ausgewählt aus der aus Alkylresten, Oxaalkylresten oder Alkenylresten mit 1 bis 10 Kohlenstoffatomen bestehenden Gruppe, aufweisen, und Oxaalkylengruppierungen der Formel -R'-(OCH₂CH₂)_{q}-OR'- oder -R'-[OCH(CH₃)CH₂]_{q}-OR'-, worin R' H oder ein unverzweigter Alkylenrest mit 1 bis 18 Kohlenstoffatomen ist und gilt: 1≤q≤22, bestehenden Gruppe ausgewählt ist.

5. Verbindung nach Anspruch 4, **dadurch gekennzeichnet, dass** die Substituenten R₁ₙ, R₂ₙ und R₃ bis R₈ unabhängig voneinander aus der aus unverzweigten Alkylresten mit 1 bis 18 Kohlenstoffatomen, 2-Ethylhexyl, Phenyl, und Oxaalkylen der Formel R-(OCH₂CH₂)_{y}- oder R-[OCH(CH₃)CH₂]_{y}-, worin R H oder ein unverzweigter Alkylrest mit 1 bis 18 Kohlenstoffatomen ist und gilt: 1≤y≤22, bestehenden Gruppe ausgewählt sind.

6. Verfahren zur Polymerisation oder Vemetzung von Monomeren oder Präpolymeren, die in der Lage sind, kationisch zu reagieren, **dadurch gekennzeichnet, dass** eine Verbindung nach Anspruch 1 als Photoinitiator-Säurequelle, die die Reaktion katalysiert, verwendet wird.

7. Verfahren nach Anspruch 6, **dadurch gekennzeichnet, dass** die Monomere aus der aus Verbindungen, die eine zyklische Etherfunktionalität, eine zyklische Thioetherfunktionalität oder eine zyklische Aminfunktionalität tragen, Vinylverbindungen, Vinylethern, Oxazolinen, Lactonen und Lactamen bestehenden Gruppe ausgewählt sind.

8. Verfahren nach Anspruch 6, **dadurch gekennzeichnet, dass** das Präpolymer aus der aus Verbindungen, in denen Epoxygruppierungen von einer aliphatischen Kette, einer aromatischen Kette oder einer heterozyklischen Kette getragen werden, bestehenden Gruppe ausgewählt ist.

9. Verfahren nach Anspruch 6, **dadurch gekennzeichnet, dass** es darin besteht, den Photoinitiator mit zumindest einem Monomer oder Präpolymer zu vermischen, das in der Lage ist, kationisch zu polymerisieren, und das erhaltene Gemisch aktinischer Strahlung oder β-Strahlung auszusetzen.

10. Verfahren nach Anspruch 9, **dadurch gekennzeichnet, dass** das Reaktionsgemisch Strahlung ausgesetzt wird, nachdem es zu einer dünnen Schicht geformt wurde.

11. Verfahren nach Anspruch 6, **dadurch gekennzeichnet, dass** die Menge des verwendeten Photoinitiators zwischen 0,01 und 15 Gew.-% des Monomer- oder Präpolymergewichts beträgt.

12. Verfahren nach Anspruch 6, **dadurch gekennzeichnet, dass** der Photoinitiator in Form einer Lösung in einem Lösungsmittel verwendet wird, das bei der Polymerisationsreaktion inert ist.

13. Verfahren nach Anspruch 12, **dadurch gekennzeichnet, dass** das inerte Lösungsmittel aus der aus Aceton, Methylethylketon, Acetonitril, Propylencarbonat, γ-Butyrolacton, Etherestern von Mono-, Di-, Triethylen- oder -propylenglykolen, Etheralkoholen von Mono-, Di-, Triethylen- oder -propylenglykolen, Phthalsäure- und Zitronensäurestern bestehenden Gruppe ausgewählt ist.

14. Verfahren nach Anspruch 6, **dadurch gekennzeichnet, dass** die Reaktion in Gegenwart eines Lösungsmittels oder eines Verdünnungsmittels durchgeführt wird, das aus einer bei der Polymerisation aktiven Verbindung besteht.

15. Verfahren nach Anspruch 14, **dadurch gekennzeichnet, dass** die reaktive Verbindung aus der aus Mono- und Divinylethern von Mono-, Di-, Tri-, Tetraethylen- oder -propylenglykolen, Trivinylether, Trimethylolpropan und Dimethanolcyclohexandivinylether, N-Vinylpyrrolidon und Propylencarbonat-2-propenylether bestehenden Gruppe ausgewählt ist.

16. Verfahren nach Anspruch 6, **dadurch gekennzeichnet, dass** dem Reaktionsmedium ein Photosensibilisator zugesetzt wird.

17. Verfahren nach Anspruch 16, **dadurch gekennzeichnet, dass** der Photosensibilisator aus der aus Anthracen, Diphenyl-9,10-anthracen, Perylen, Phenothiazin, Tetracen, Xanthon, Thioxanthon, Isopropylthioxanton, Acetophenon, Benzophenon, 1,3,5-Triaryl 2-pyrazolinen und Derivaten davon, insbesondere Derivaten mit Substitutionen an den aromatischen Ringen in Form von Alkyl-, Oxaalkyl- oder Azaalkylresten, bestehenden Gruppe ausgewählt ist.

18. Verfahren nach Anspruch 6, **dadurch gekennzeichnet, dass** das Reaktionsgemisch weiters zumindest ein Monomer oder Präpolymer, das zu radikalischer Polymerisation in der Lage ist, und eine Verbindung, die in der Lage ist, einen radikalischen Polymerisationsinitiator unter der Einwirkung von aktinischer Strahlung oder β-Strahlung oder unter Wärmeeinfluss freizusetzen, enthält.

19. Verfahren zur Modifizierung der Löslichkeitseigenschaften eines Polymers, das säureempfindliche Gruppierungen aufweist, **dadurch gekennzeichnet, dass** es darin besteht, das Polymer in Gegenwart einer Verbindung nach Anspruch 1 aktinischer Strahlung oder β-Strahlung auszusetzen.

20. Verfahren nach Anspruch 19, **dadurch gekennzeichnet, dass** das Polymer Estermotive, oder Arylethermotive von tertiärem Alkohol enthält.

21. Verfahren nach Anspruch 20, **dadurch gekennzeichnet, dass** das Polymer aus der aus tert-Butylpolyacrylaten, tert-Butylpolyitaconaten, Poly(tert-butoxycarbonyloxystyrol) und Poly(tert-butoxystyrol) bestehenden Gruppe ausgewählt ist.

22. Verfahren nach Anspruch 19, **dadurch gekennzeichnet, dass** es zur chemischen Verstärkung von Photoresists durchgeführt wird.

## Claims

1. Ionic compound comprising at least one group A⁺X⁻, **characterized in that**:
- A⁺ is a cationic group selected from the group consisting of biaryliodonium, arylsulfonium, arylacylsulfonium, diazonium groups and organometallic cations comprising a transition metal complexed with at least one unsaturated cycle comprising 4 to 12 carbon atoms, said cationic group possibly being part of a polymer chain;
- X⁻ is an imide anion [FSO₂NSO₂R'_{F}]⁻ or [R_{F}CH₂OSO₂NSO₂R'_{F}]⁻ or [(R_{F})₂CHOSO₂NSO₂R'_{F}]⁻, or a methylide anion [FSO₂C(Q)SO₂R'_{F}]⁻ or [R_{F}CH₂OSO₂C(Q)SO₂R'_{F}]⁻ or [(R_{F})₂CHOSO₂C(Q) SO₂R'_{F}]⁻ in which:
1) Q represents:
- H-, Cl-, F-, Br- or CN- ;
- an alkyl radical having 1 to 30 carbon atoms;
- an aryl or alkylaryl or arylalkyl radical having 6 to 30 carbon atoms;
- a group R"_{F}-, a group R"_{F}SO₂-, a group R"_{F}CH₂O-SO₂- or a group (R"_{F})₂CHO-SO₂- ;
2) R_{F} and R'_{F}, as well as R"_{F} when X⁻ is a methylide anion, are independently selected from the group consisting of fluorine, perhaloalkyl groups having 1 to 30 carbon atoms, (perhaloalkyl)alkoxy groups having 2 to 30 carbon atoms, perhalogenated cycloaliphatic groups having 3 to 30 carbon atoms possibly containing heteroatoms selected from O and N and/or possibly carrying at least one perhaloalkyl chain, and perhalogenated aryl groups having 6 to 30 carbon atoms; or
3) R_{F} and R'_{F} together form a bivalent radical selected from perfluorinated linear alkylene radicals having 2 to 8 carbon atoms; or
4) when X⁻ is a methylide anion, R'_{F} and R"_{F} on the one hand, or R_{F} and R"_{F} on the other hand together form a bivalent radical selected from perfluorinated linear alkylene radicals having 2 to 8 carbon atoms.

2. Compound according to claim 1, **characterized in that** the anion is a sulfonimide anion having one of the following formulae [FSO₂NSO₂R'_{F}]⁻, [CF₃CH₂OSO₂NSO₂R'_{F}]⁻ and [(CF₃)₂CHOSO₂NSO₂R'_{F}]⁻, or a sulfonylmethylide anion having one of the formulae [FSO₂C(Q)SO₂R'_{F}]⁻, [CF₃CH₂OSO₂C(Q)SO₂R'_{F}]⁻ or [(CF₃)₂CHOSO₂-C(Q)SO₂R'_{F}]⁻, in which R'_{F} is F, CF₃CH₂O-, (CF₃)₂CH-O- or a perfluoroalkyl group having 1 to 10 carbon atoms and Q is selected from the group consisting of hydrogen, FSO₂-, CF₃CH₂O-SO₂-, (CF₃)₂CH-O-SO₂-, alkyl, aryl, alkylaryl or arylakyl groups having at most 30 carbon atoms, perfluoroalkylsulfonyl groups having 1 to 8 carbon atoms, perfluoroalkyl groups having 1 to 12 carbon atoms; or Q and R'_{F} together form a bivalent perfluorinated linear alkylene group having 1 to 8 carbon atoms.

3. Compound according to claim 1, **characterized in that** the anion is selected from the group consisting of (FSO₂)₂N⁻, (FSO₂)₃C⁻, (FSO₂)₂CH⁻, (CF₃CH₂OSO₂)₂N⁻, [(CF₃)₂CHOSO₂]₂N⁻, (CF₃CH₂OSO₂)₂CH⁻, [(CF₃)₂CHOSO₂]₂CH⁻, [(CF₃)₂CHOSO₂]₃C⁻, [(CF₃)₂CHOSO₂]₃C⁻, [FSO₂NSO₂CF₃]⁻, [FSO₂NSO₂C₂F₅]⁻, [(CF₃)₂CHO-SO₂NSO₂CF₃]⁻, [(CF₃)₂CHOSO₂NSO₂CF₃]⁻, [(CF₃)₂CHOSO₂NSO₂C₂F₅]⁻ and [(CF₃CH₂OSO₂NSO₂C₂F₅]⁻.

4. Compound according to claim 1, **characterized in that** the cation A⁺ has the formula 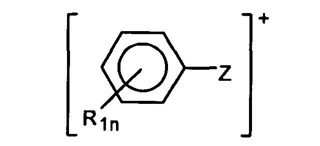 in which Z is selected from the group consisting of:
- aryliodonium groups 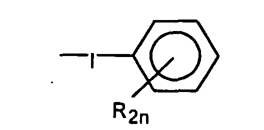
- sulfonium groups 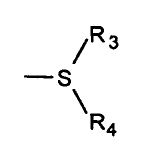
- acylsulfonium groups 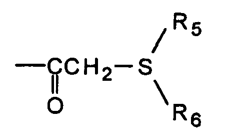
- diazonium groups
N≡N-R₈
- organometallic groups 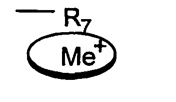 and in which R₁ₙ represents 1 to 4, preferably 1 to 2 groups which are identical or different and are bound to any free carbon atom of the aryl group, R₂ₙ represents 1 to 4, preferably 1 to 2 groups which are identical or different and are bound to any free carbon atom of the aryl group, and groups R₁ₙ, R₂ₙ, and R₃ to R₈ are independently selected from:
• linear or branched alkyl or arylalkyl radicals having 1 to 30 carbon atoms;
• alkenyl radicals having 1 to 30 carbon atoms;
• aryl or alkylaryl radicals having 6 to 30 carbon atoms, including those which have condensed nuclei;
• radicals having 1 to 30 carbon atoms and selected from the group consisting of oxaalkyls, azaalkyls, thiaalkyls, phosphaalkyls, oxaalkylenes, azaalkylenes, thiaalkylenes, phosphaalkylenes,
• radicals having 1 to 30 carbon atoms including a sulfoxide, sulfone, phosphine oxide, phosphonate group, all these radicals being obtained by addition of oxygen on sulfur or phosphorus atoms;
• aromatic or alicyclic heterocyclic radicals comprising at least one heteroatom selected from the group consisting of O, N, S and P ;
• -NO, -CN, -OH, -Cl, -Br, -I, -F ;
• or two substituents selected from R₁ₙ and/or two substituents selected from R₂ₙ, or substituents R₃ and R₄, or substituents R₅ and R₆ together form a bivalent radical which form a cycle with the group carrying it, said bivalent radical being selected from the group consisting of linear alkylene radicals having 1 to 18 carbon atoms, benzo biradicals possibly carrying at least one substituent preferably selected from the group consisting of alkyl, oxaalkyl or alkenyl radicals having 1 to 10 carbon atoms, oxaalkylene groups having the formula -R'-(OCH₂CH₂)_{q}-OR- or -R'-[OCH(CH₃)CH₂]_{q}-OR'- in which R' is H or a linear alkylene radical having 1 to 18 carbon atoms and 1≤q≤22.

5. Compound according to claim 4, **characterized in that** the substituents R₁ₙ, R₂ₙ, and R₃ to R₈ are independently selected from the group consisting of linear alkyl radicals having 1 to 18 carbon atoms, 2-ethylhexyl, phenyl, oxaalkyls having formula R-(OCH₂CH₂)_{y}- or R-[OCH(CH₃)CH₂]_{y}- in which R is H or a linear alkyl radical having 1 to 18 carbon atoms and 1≤y≤22.

6. Process for the polymerization or cross-linking of monomers or prepolymers capable of cationic reaction, **characterized in that** there is used, a compound according to claim 1 as photoinitiator constituting a source of acid catalyzing the reaction.

7. Process according to claim 6, **characterized in that** the monomers are selected from the group consisting of compounds which include a cyclic ether function, a cyclic thioether function, a cyclic amine function, vinyl compounds, vinyl ethers, oxazolines, lactones and lactames.

8. Process according to claim 6, **characterized in that** the prepolymer is selected from the group consisting of compounds in which epoxy groups are carried by an aliphatic chain, an aromatic chain or a heterocyclic chain.

9. Process according to claim 6, **characterized in that** it consists in mixing the photoinitiator with at least one monomer or prepolymer capable of cationic polymerization, and subjecting the mixture obtained to actinic or β-radiation.

10. Process according to claim 9, **characterized in that** the reaction mixture is subject to radiation after having been formed into a thin layer.

11. Process according to claim 6, **characterized in that** the quantity of photoinitiator used is between 0.01 and 15% by weight with respect to weight of the monomer or prepolymer.

12. Process according to claim 6, **characterized in that** the photoinitiator is used in the form of a solution in a solvent which is inactive towards the polymerization reaction.

13. Process according to claim 12, **characterized in that** the inactive solvent is selected from the group consisting of acetone, methyl-ethyl ketone, acetonitrile, propylene carbonate, γ-butyrolactone, ether-esters of mono-, di-, triethylene or propylene glycols, ether-alcohols of mono-, di-, triethylene or propylene glycols, esters of phtalic acid or citric acid.

14. Process according to claim 6, **characterized in that** the reaction is carried out in the presence of a solvent or a diluent consisting of a compound which is reactive towards the polymerization.

15. Process according to claim 14, **characterized in that** the reactive compound is selected from the group consisting of vinyl mono- and diethers of mono-, di-, tri-, tetratethylene or propylene glycols, trivinyl ether trimethylopropane, and divinylether of dimethanolcyclohexane, N-vinylpyrolidone, 2-propenylether of propylene carbonate.

16. Process according to claim 6, **characterized in that** a photosensitizer is added to the reaction mixture.

17. Process according to claim 16, **characterized in that** the photosensitizer is selected from the group consisting of anthracene, diphenyl-9, 10-anthracene, perylene, phenothiazine, tetracene, xanthone, thioxanthone, isopropylthioxanthone, acetophenone, benzophenone, 1,3,5-triaryl-2-pyrazolines and derivatives thereof, in particular derivatives substituted on the aromatic nuclei by means of alkyl, oxa-, or azaalkyl radicals.

18. Process according to claim 6, **characterized in that** the reaction mixture additionally contains at least one monomer or prepolymer capable of free radical polymerization and a compound capable of releasing an initiator of free radical polymerization under actinic or β-radiation or under heat.

19. Process for modifying the solubility properties of a polymer having groups which are sensitive towards acids, **characterized in that** it consists in subjecting said polymer to actinic radiation or β-radiation, in the presence of a compound according to claim 1.

20. Process according to claim 19 **characterized in that** the polymer contains ester units or arylether of tertiary alcohol units.

21. Process according to claim 20, **characterized in that** the polymer is selected from the group consisting of tertiobutyl polyacrylates, tertiobutyl polyitaconates, poly(tertiobutoxycarbonyloxystyrene), poly(tertiobutoxystyrene).

22. Process according to claim 19, **characterized in that** it is implemented for chemical amplification of photoresists.
